# EUROPEAN PATENT SPECIFICATION

(11) **EP 1 448 559 B1**
(45) Date of publication and mention of the grant of the patent: **27.12.2006**
(21) Application number: 02779711.7
(22) Date of filing: 21.11.2002
(51) Int. Cl.: C07D 417/12, A61K 31/4439, A61P 5/48

(54) **5-(4-(2-(N-METHYL-N-(2-PYRIDYL)AMINO)ETHOXY)BENZYL)THIAZOLIDINE-2,4-DIONE BENZENESULFONATE; PROCESS FOR ITS PREPARATION; POLYMORPHS I, II AND III THEREOF; AND ITS USE AS PHARMACEUTICAL ACTIVE INGREDIENT**
5-(4-(2-(N-METHYL-N-(2-PYRIDYL)AMINO)ETHOXY)BENZYL)THIAZOLIDINE-2,4-DION-BENZOLSULFONAT; PROZESS ZU SEINER HERSTELLUNG; POLYMORPHE I, II UND III DAVON; UND SEINE VERWENDUNG ALS PHARMAZEUTISCHER WIRKSTOFF
5-(4-(2-(N-METHYL-N-(2-PYRIDIL)AMINO)ETHOXY)BENZYL)THIAZOLIDINE-2,4-DIONE BENZENESULFONATE, PROCEDE DE PREPARATION, POLYMORPHES I, II ET III DE CELUI-CI ET SON UTILISATION COMME INGREDIENT ACTIF

(30) Priority: 21.11.2001 GB 0127934; 21.11.2001 GB 0127935; 21.11.2001 GB 0127936; 21.11.2001 GB 0127937
(43) Date of publication of application: 25.08.2004
(73) Proprietor: SMITHKLINE BEECHAM PLC, Brentford, Middlesex TW8 9GS (GB)
(72) Inventor: Craig, Andrew, Simon, Tonbridge, Kent TN11 9AN (GB); Millan, Michael, John, Tonbridge, Kent TN11 9AN (GB)
(74) Representative: Rutter, Keith
(86) International application number: PCT/GB2002/005232
(87) International publication number: WO 2003/045946

(56) References cited:
- WO-A-00/63205
- WO-A-00/63206
- WO-A-01/68646
- WO-A-02/20520
- WO-A-94/05659
- WO-A-02/051839
- BERGE S M ET AL: "PHARMACEUTICALS SALTS" JOURNAL OF PHARMACEUTICAL SCIENCES, AMERICAN PHARMACEUTICAL ASSOCIATION. WASHINGTON, US, vol. 66, no. 1, 1977, pages 1-19, XP000562636 ISSN: 0022-3549
- BASTIN, R. J. ET AL.: "Salt Selection and Optimisation for Pharmaceutical New Chemical Entities" ORG. PROC. RES. & DEV., vol. 4, no. 5, 2000, pages 427-435, XP002228592

## Description

This invention relates to a novel compound, in particular to a novel pharmaceutical being a novel salt of a certain thiazolidinedione, to a process for the preparation of the said compound and to the use of the compound in medicine.

EP-A-0 306 228 relates to certain thiazolidinedione derivatives disclosed as having hypoglycaemic and hypolipidaemic activity. The compound of Example 30 of EP-A-0 306 228 is 5-[4-[2-(N-methyl-N- (2-pyridyl)amino) ethoxy]benzyl]thiazolidine-2,4-dione (hereinafter referred to as "Compound (I)").

WO 94/05659 discloses certain salts of the compounds of EP-A-0 306 228. The preferred salt of WO 94/05659 is the maleic acid salt.

We have now prepared and characterised a benzenesulfonate salt of Compound (I) (hereinafter also referred to as the "Benzenesulfonate") and discovered that a novel form of the benzenesulfonate salt is formed that is particularly stable and hence is suitable for bulk preparation and handling. The Benzenesulfonate also has a high melting point and possesses good bulk flow properties. The Benzenesulfonate is therefore surprisingly amenable to large scale pharmaceutical processing and especially to large scale milling.

The novel salt can also be prepared by an efficient and economic process particularly suited to large-scale preparation.

The novel benzenesulfonate also has useful pharmaceutical properties and in particular it is indicated to be useful for the treatment and/or prophylaxis of diabetes mellitus, conditions associated with diabetes mellitus and certain complications thereof.

It has been found that the Benzenesulfonate exists in more than one polymorphic form. The present invention extends to certain polymorphic forms whether in a pure polymorphic form or when admixed with any other material, such as another polymorphic form. Herein, the novel polymorpic forms of the Benzenesulfonate are referred to as Form I, Form II and Form III.

Accordingly, the present invention provides a 5-[4-[2-(N-methyl-N-(2-pyridyl)amino)etboxy]benzyl]thiazolidine-2,4-dione benzenesulfonate salt (the Benzenesulfonate) or a solvate thereof wherein the Benzene sulphonate is Form II, Form III or propan-2-ol solvate.

Suitably, the invention provides the Benzenesulfonate Form II, Form III or propan-2-ol solvate characterised by data provided by at least one of the following: spectral data including infra red, Raman, X-ray, carbon or hydrogen nuclear magnetic resonance spectral data and melting point data.

In one aspect the invention provides Benzenesulfonate Form II.

In a further aspect the invention provides Benzenesulfonate Form III.

In a particular aspect, the present invention provides 5-[4-[2-(N-methyl-N-(2-pyridyl)amino)ethoxy]benzyl]thiazolidine-2,4-dione benzenesulfonate salt, Form II, characterised by
(i) an infra red spectrum containing peaks at about 1646, 837, 565 and 523 cm⁻¹; and/or
(ii) a Raman spectrum containing peaks at about 1695, 1550 and 664 cm⁻¹; and/or
(iii) an X-ray powder diffraction (XRPD) pattern containing peaks at about 6.3, 8.1, 10.3, 12.3 and 14.2 °2θ.

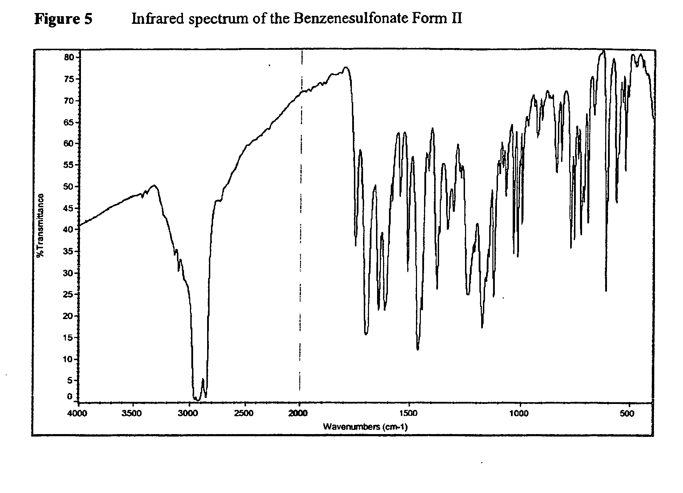

In one favoured aspect, the Benzenesulfonate Form II provides an infrared spectrum substantially in accordance with Figure 5.

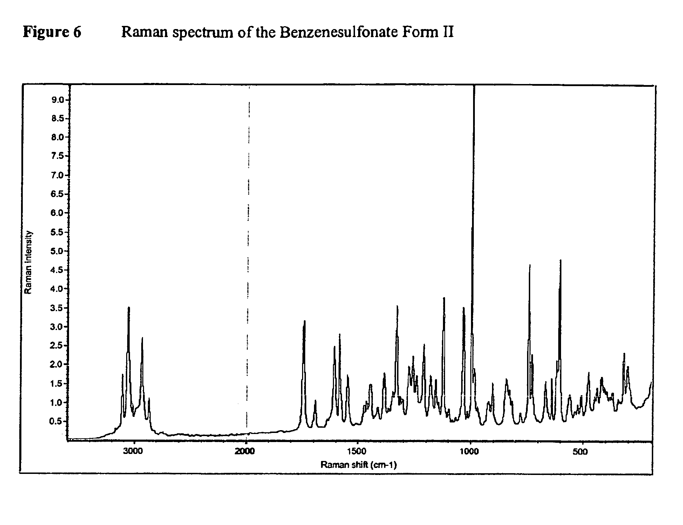

In one favoured aspect, the Benzenesulfonate Form II provides a Raman spectrum substantially in accordance with Figure 6.

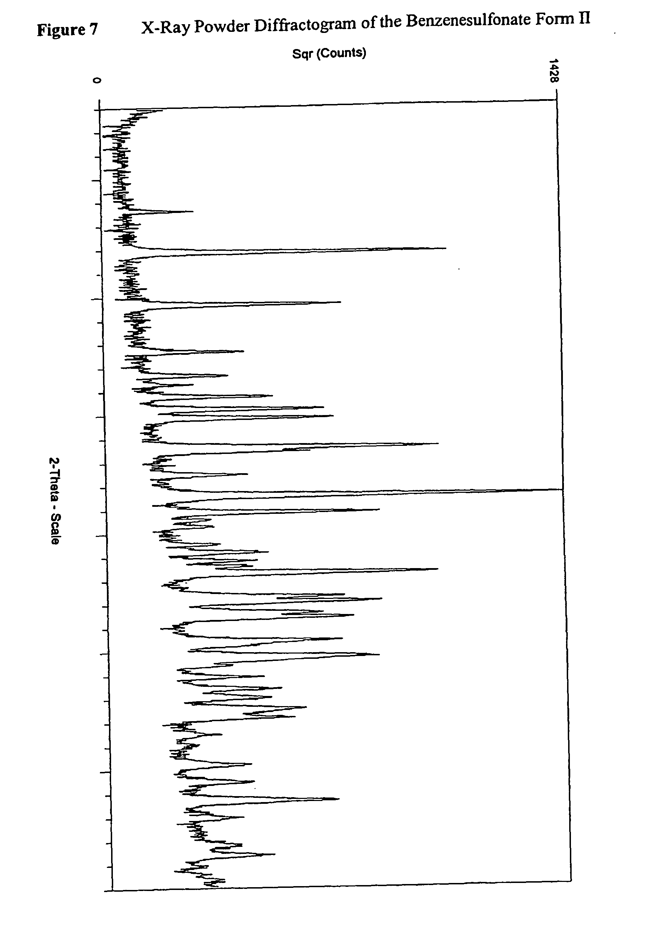

In one favoured aspect, the Benzenesulfonate Form II provides an X-Ray powder diffraction pattern (XRPD) substantially in accordance with Table 2 or Figure 7.

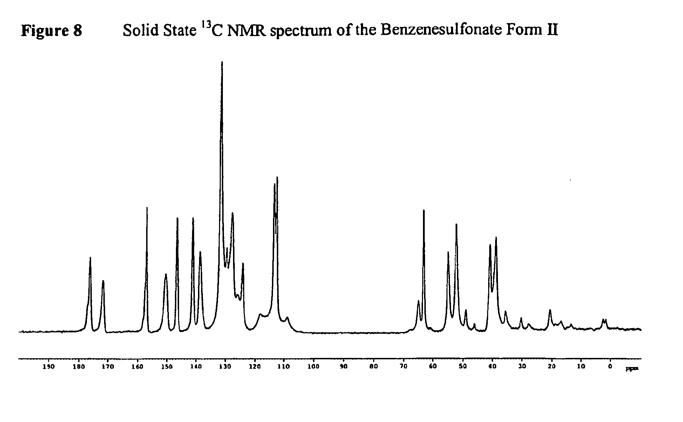

In one favoured aspect, the Benzenesulfonate Form II provides a Solid State ¹³C NMR spectrum substantially in accordance with Figure 8.

In a further favoured aspect the Benzenesulfonate Form II, provides a melting range in the range of 175-185 °C, for example 177.6-180.3°C.

In a most preferred aspect, the invention provides 5-[4-[2-(N-methyl-N-(2-pyridyl)amino)ethoxy]benzyl]thiazolidine-2,4-dione, benzenesulfonate salt Form II, characterised in that it provides:
(i) an infrared spectrum substantially in accordance with Figure 5; and
(ii) a Raman spectrum in accordance with Figure 6; and
(iii) an X-Ray powder diffraction pattern (XRPD) in accordance with Table 2 or Figure 7; and
(iv) a Solid State ¹³C NMR spectrum in accordance with Figure 8.

In a particular aspect, the present invention provides 5-[4-[2-(N-methyl-N-(2-pyridyl)amino)ethoxy]benzyl]thiazolidine-2,4-dione benzenesulfonate salt, Form III, characterised by
(i) an infra red spectrum containing peaks at 1313, 1163, 706, 568 and 519 cm⁻¹; and/or
(ii) a Raman spectrum containing peaks at 2920, 1442, 1207, 1144, 658 and 308 cm⁻¹; and/or
(iii) an X-ray powder diffraction (XRPD) pattern containing peaks at 11.5, 12.0, 13.1, 17.8 and 18.3 °2θ.

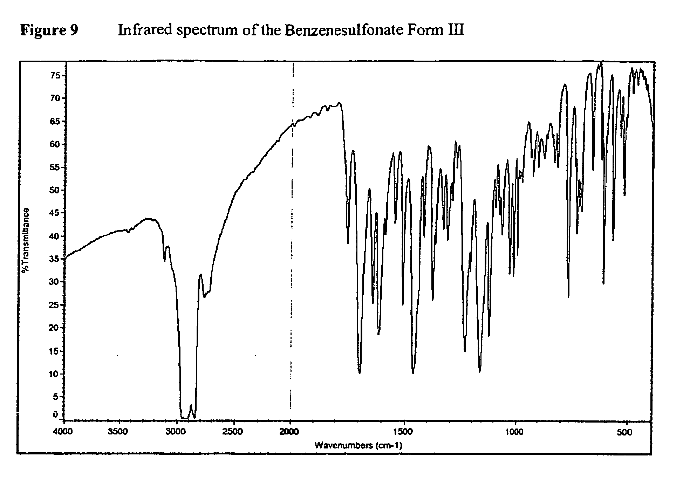

In one favoured aspect, the Benzenesulfonate Form III provides an infrared spectrum in accordance with Figure 9.

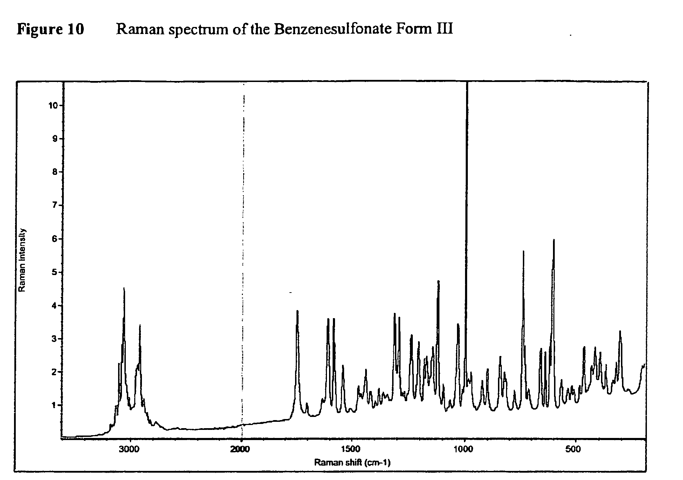

In one favoured aspect, the Benzenesulfonate Form III provides a Raman spectrum in accordance with Figure 10.

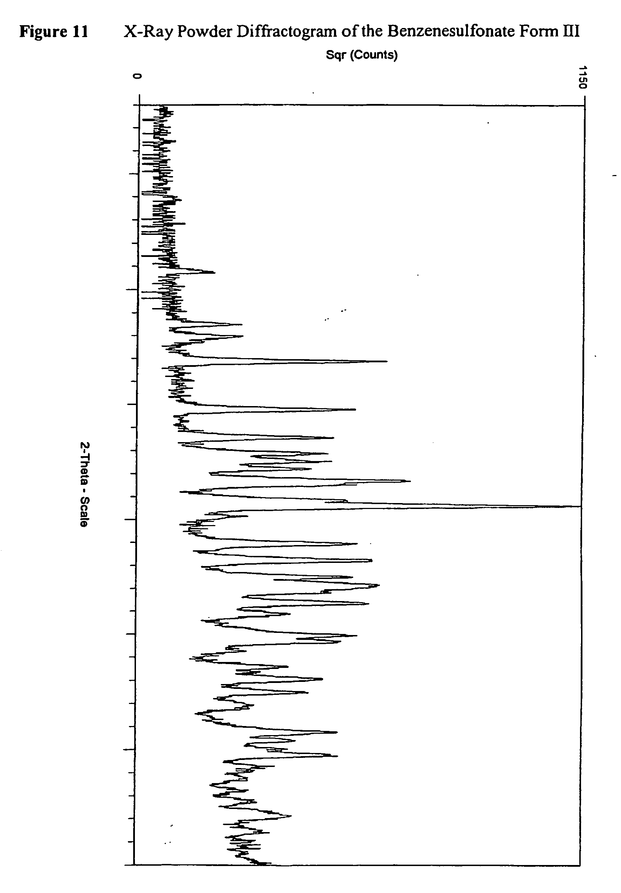

In one favoured aspect, the Benzenesulfonate Form III provides an X-Ray powder diffraction pattern (XRPD) in accordance with Table 3 or Figure 11.

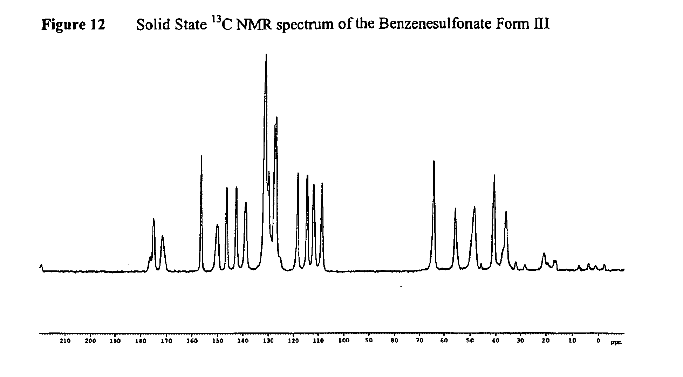

In one favoured aspect, the Benzenesulfonate Form III provides a Solid State ¹³C NMR spectrum in accordance with Figure 12.

In a further favoured aspect the Benzenesulfonate Form III, provides a melting range in the range of 173-183 °C, for example 176.9-177.3°C.

In a most preferred aspect, the invention provides 5-[4-[2-(N-methyl-N-(2-pyridyl)amino)ethoxy]benzyl]thiazolidine-2,4-dione, benzenesulfonate salt Form III, characterised in that it provides:
(i) an infrared spectrum in accordance with Figure 9; and
(ii) a Raman spectrum in accordance with Figure 10; and
(iii) an X-Ray powder diffraction pattern (XRPD) in accordance with Table 3 or Figure 11; and
(iv) a Solid State ¹³C NMR spectrum in accordance with Figure 12.

As indicated the benzenesulfonate forms solvates. Suitable solvates are pharmaceutically acceptable solvates, including hydrates.

In a further particular aspect the invention provides 5-[4-[2-(N-methyl-N-(2-pyridyl)amino)ethoxy]benzyl]thiazolidine-2,4-dione benzenesulfonate salt propan-2-ol solvate (the Benzenesulphonate propan-2-ol solvate), characterised in that it provides:
(i) an infra red spectrum containing a peak at 3541 cm⁻¹; and/or
(ii) a Raman spectrum containing peaks at 893 and 317 cm⁻¹; and/or
(iii) an X-ray powder diffraction (XRPD) pattern containing peaks at 9.5, 13.3, 20.1 and 26.8 °2θ.

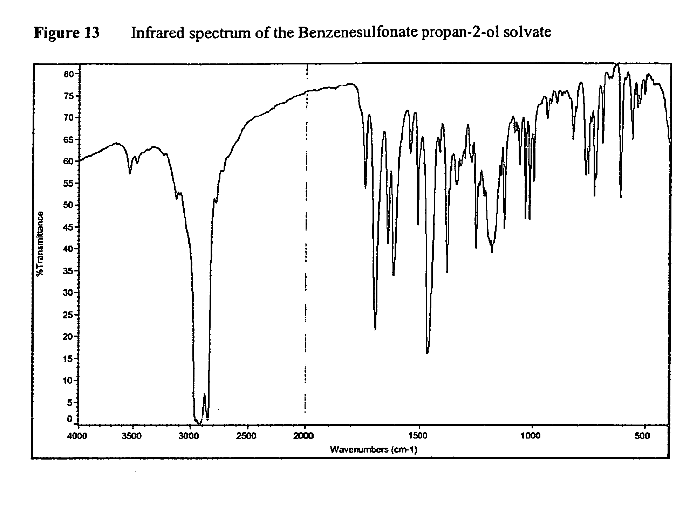

In one favoured aspect, the Benzenesulphonate propan-2-ol solvate provides an infrared spectrum in accordance with Figure 13.

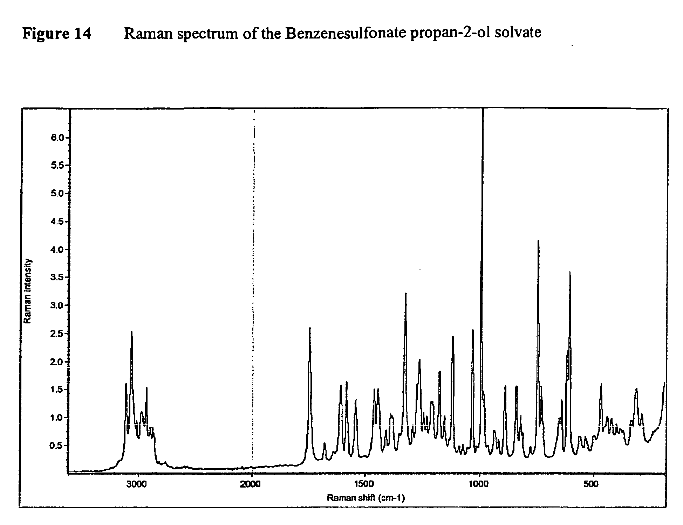

In one aspect, the Benzenesulphonate propan-2-ol solvate provides a Raman spectrum in accordance with Figure 14.

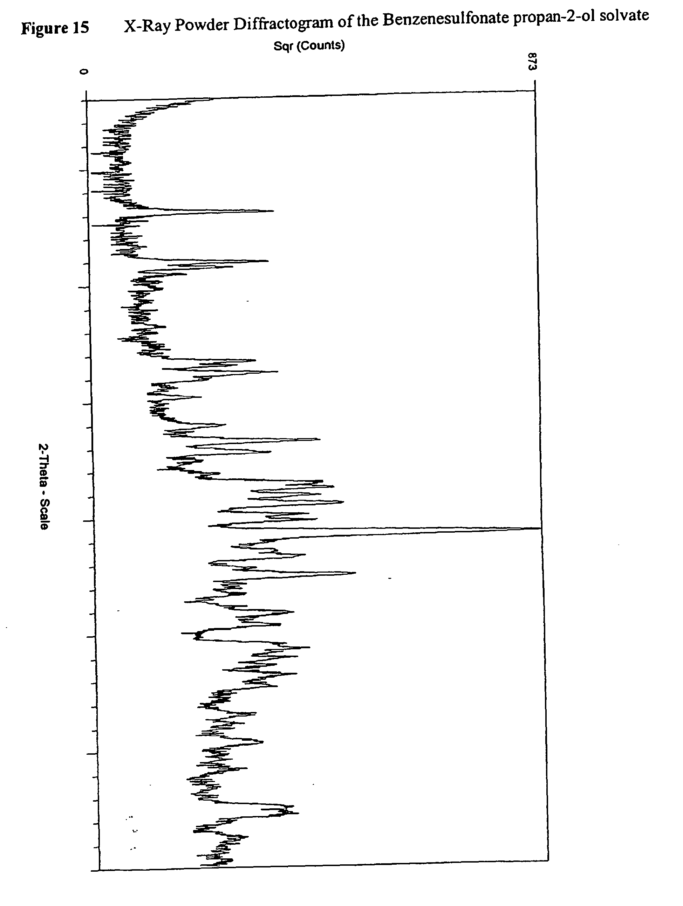

In one aspect, the Benzenesulphonate propan-2-ol solvate provides an X-Ray powder diffraction pattern (XRPD) in accordance with Table 4 or Figure 15.

In one aspect, the Benzenesulphonate propan-2-ol solvate provides, provides a melting range in the range of 176-184 °C, for example 180.4-180.7°C.

The present invention encompasses the Benzenesulphonate or a solvate thereof isolated in a purified form or when admixed with other materials.

Thus in one aspect there is provided the Benzenesulphonate or a solvate thereof in an isolated form, that is isolated substantially from any impurity. Thus in a particular aspect there is provided the Benzenesulphonate or a solvate thereof in substantially pure form.

In yet a further aspect there is provided the Benzenesulphonate or a solvate thereof in crystalline form.

Also, the invention provides the Benzenesulfonate or solvate thereof in a solid pharmaceutically acceptable form, such as a solid dosage form, especially when adapted for oral administration.

Moreover, the invention also provides the Benzenesulfonate or a solvate thereof in a pharmaceutically acceptable form, especially in bulk form, such form being particularly capable of being milled. The invention therefor also provides the Benzenesulfonate or a solvate thereof in a milled form.

Furthermore, the invention provides the Benzenesulfonate or a solvate thereof in a pharmaceutically acceptable form, especially in bulk form, such form having good flow properties, especially good bulk flow properties.

The invention also provides a process for preparing the Benzenesulfonate, characterised in that 5-[4-[2-(N-methyl-N-(2-pyridyl)amino)ethoxy]benzyl]thiazolidine-2,4-dione (Compound (I))or a salt thereof, preferably dispersed or dissolved in a suitable solvent, is reacted with a suitable source of benzenesulfonate ion, and the Benzenesulfonate or a solvate thereof is recovered.

A suitable reaction solvent is an alkanol, for example propan-2-ol, or a hydrocarbon, such as toluene, a ketone, such as acetone, an ester, such as ethyl acetate, an ether such as tetrahydrofuran, a nitrile such as acetonitrile, or a halogenated hydrocarbon such as dichloromethane or water, or an organic acid such as acetic acid; or a mixture thereof.

Conveniently, the source of benzenesulfonate ion is benzenesulfonic acid. The benzenesulfonic acid may be added as a solid or in solution, for example in water, ether, ketone, nitrile or a lower alcohol such as methanol, ethanol, or propan-2-ol, or a mixture of solvents.

For example, a solution of benzenesulfonic acid in propan-2-ol or tetrahydrafuran (THF) may be added to a solution of Compound (I) in the same solvent.

An alternative source of benzenesulfonate ion is provided by a base salt of benzenesulfonic acid for example ammonium benzenesulfonate, or the benzenesulfonic acid salt of an amine, for example ethylamine or diethylamine.

The concentration of Compound (I) is preferably in the range 3 to 50% weight/volume, more preferably in the range 5 to 20%. The concentration of benzenesulfonic acid solutions are preferably in the range of 5 to 75% weight/volume.

The reaction is usually carried out at ambient temperature or at an elevated temperature, for example at the reflux temperature of the solvent, although any convenient temperature that provides the required product may be employed.

Solvates, such as hydrates, of the Benzenesulfonate may be prepared according to conventional procedures, for example by crystallising or recrystallising from a solvent which provides or contains the solvate moiety, or by exposing the Benzenesulfonate to the solvate moiety as a vapour.

Recovery of the required compound generally comprises crystallisation from an appropriate solvent, conveniently the reaction solvent, usually assisted by cooling. For example, the Benzenesulfonate may be crystallised from an ether such as tetrahydrofuran or an alkanol such as propan-2-ol. An improved yield of the salt can be obtained by evaporation of some or all of the solvent or by crystallisation at elevated temperature followed by controlled cooling. Careful control of precipitation temperature and seeding may be used to improve the reproducability of the product form.

Crystallisation can also be initiated by seeding with crystals of the Benzenesulfonate but this is not essential.

In our hands either Benzenesulfonate Form I, Form II or Form III or mixtures thereof is produced by the above mentioned process. To ensure formation of a particular polymorph in substantially pure form the crystallisation step is preferably seeded with seeds of the required polymorph. Accordingly, Benzenesulfonate Form I is suitably crystallised by seeding with seeds of Benzenesulfonate Form I. Benzenesulfonate Form II is suitably crystallised by seeding with seeds of Benzenesulfonate Form II. Benzenesulfonate Form III is suitably crystallised by seeding with seeds of Benzenesulfonate Form III.

Compound (I) is prepared according to known procedures, such as those disclosed in EP-A-0 306 228 and WO 94/05659. The disclosures of EP-A-0 306 228 and WO 94/05659 are incorporated herein by reference.

Benzenesulfonic acid is a commercially available compound.

When used herein the term "Tₒₙₛₑₜ" is generally determined by Differential Scanning Calorimetry and has a meaning generally understood in the art, as for example expressed in Pharmaceutical Thermal Analysis, Techniques and Applications", Ford and Timmins, 1989 as "The temperature corresponding to the intersection of the pre-transition baseline with the extrapolated leading edge of the transition".

When used herein in respect of certain compounds the term "good flow properties" is suitably characterised by the said compound having a Hausner ratio of less than or equal to 1.5, especially of less than or equal to 1.25.

"Hausner ratio" is an art accepted term.

When used herein the term 'prophylaxis of conditions associated with diabetes mellitus' includes the treatment of conditions such as insulin resistance, impaired glucose tolerance, hyperinsulinaemia and gestational diabetes.

Diabetes mellitus preferably means Type II diabetes mellitus.

Conditions associated with diabetes include hyperglycaemia and insulin resistance and obesity. Further conditions associated with diabetes include hypertension, cardiovascular disease, especially atherosclerosis, certain eating disorders, in particular the regulation of appetite and food intake in subjects suffering from disorders associated with under-eating, such as anorexia nervosa, and disorders associated with over-eating, such as obesity and anorexia bulimia. Additional conditions associated with diabetes include polycystic ovarian syndrome and steroid induced insulin resistance.

The complications of conditions associated with diabetes mellitus encompassed herein includes renal disease, especially renal disease associated with the development of Type II diabetes including diabetic nephropathy, glomerulonephritis, glomerular sclerosis, nephrotic syndrome, hypertensive nephrosclerosis and end stage renal disease.

As mentioned above the compound of the invention has useful therapeutic properties: The present invention accordingly provides the Benzenesulfonate for use as an active therapeutic substance.

More particularly, the present invention provides the Benzenesulfonate for use in the treatment and/or prophylaxis of diabetes mellitus, conditions associated with diabetes mellitus and certain complications thereof.

The Benzenesulfonate may be administered per se or, preferably, as a pharmaceutical composition also comprising a pharmaceutically acceptable carrier. Suitable methods for formulating the Benzenesulfonate are generally those disclosed for Compound (I) in the above mentioned publications.

Accordingly, the present invention also provides a pharmaceutical composition comprising the Benzenesulfonate and a pharmaceutically acceptable carrier therefor.

The Benzenesulfonate is normally administered in unit dosage form.

The active compound may be administered by any suitable route but usually by the oral or parenteral routes. For such use, the compound will normally be employed in the form of a pharmaceutical composition in association with a pharmaceutical carrier, diluent and/or excipient, although the exact form of the composition will naturally depend on the mode of administration.

Compositions are prepared by admixture and are suitably adapted for oral, parenteral or topical administration, and as such may be in the form of tablets, capsules, oral liquid preparations, powders, granules, lozenges, pastilles, reconstitutable powders, injectable and infusable solutions or suspensions, suppositories and transdermal devices. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl benzenesulfonate.

Solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the active compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the active compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

As is common practice, the compositions will usually be accompanied by written or printed directions for use in the medical treatment concerned.

As used herein the term 'pharmaceutically acceptable' embraces compounds, compositions and ingredients for both human and veterinary use: for example the term 'pharmaceutically acceptable salt' embraces a veterinarily acceptable salt.

The present invention further provides a method for the treatment and/or prophylaxis of diabetes mellitus, conditions associated with diabetes mellitus and certain complications thereof, in a human or non-human mammal which comprises administering an effective, non-toxic, amount of the Benzenesulfonate to a human or non-human mammal in need thereof.

The compositions are formulated according to conventional methods, such as those disclosed in standard reference texts, for example the British and US Pharmacopoeias, Remington's Pharmaceutical Sciences (Mack Publishing Co.), Martindale The Complete Drug Reference (London, The Pharmaceutical Press) and Harry's Cosmeticology (Leonard Hill Books).

Conveniently, the active ingredient may be administered as a pharmaceutical composition hereinbefore defined, and this forms a particular aspect of the present invention.

In a further aspect the present invention provides the use of the Benzenesulfonate for the manufacture of a medicament for the treatment and/or prophylaxis of diabetes mellitus, conditions associated with diabetes mellitus and certain complications thereof.

In the treatment and/or prophylaxis of diabetes mellitus, conditions associated with diabetes mellitus and certain complications thereof, the Benzenesulfonate may be taken in amounts so as to provide Compound (I) in suitable doses, such as those disclosed in EP-A-0 306 228, WO 94/05659 or WO 98/55122.

The unit dose compositions of the invention comprise the Benzenesulfonate in an amount providing up to 12mg, including 1-12mg such as 2-12mg of Compound (I), especially 2-4mg, 4-8mg or 8-12mg of Compound (I), for example 1, 2, 3, 4, 8, 4 to 8 or 8 to 12mg of Compound (I). Thus in particular there is provided a pharmaceutical composition comprising the Benzenesulfonate or a solvate thereof and a pharmaceutically acceptable carrier therefor, wherein the Benzenesulfonate or a pharmaceutically acceptable solvate thereof is present in an amount providing 1, 2, 3, 4, 8, 4 to 8 or 8 to 12mg of Compound (I); such as 1 mg of Compound (I); such as 2mg of Compound (I); such as 3mg of Compound (1); such as 4 of Compound (I); such as 8mg of Compound (I); such as 12mg of Compound (I);

The invention also provides a pharmaceutical composition comprising the Benzenesulfonate or a pharmaceutically acceptable solvate thereof in combination with one or more other medicaments such as anti-diabetic agents and optionally a pharmaceutically acceptable carrier therefor.

The invention also provides a method for a method for the treatment and/or prophylaxis of diabetes mellitus, conditions associated with diabetes mellitus and certain complications thereof, in a human or non-human mammal which comprises administering an effective, non-toxic, amount of the Benzenesulfonate or a pharmaceutically acceptable solvate thereof in combination with one or more other anti-diabetic agents.

In a further aspect the present invention provides the use of the Benzenesulfonate or a pharmaceutically acceptable solvate thereof in combination with one or more other anti-diabetic agents, for the manufacture of a medicament for the treatment and/or prophylaxis of diabetes mellitus, conditions associated with diabetes mellitus and certain complications thereof.

In the above mentioned treatments the administration of the Benzenesulfonate or a pharmaceutically acceptable solvate thereof and the other anti-diabetic agent or agents includes co-administration or sequential administration of the active agents.

Suitably in the above mentioned compositions, including unit doses, or treatments the Benzenesulfonate or a pharmaceutically acceptable solvate thereof is present in an amount providing up to 12mg, including 1-12mg, such as 2-12mg of Compound (I), especially 2-4mg, 4-8mg or 8-12mg of Compound (I), for example 1, 2, 3, 4, 4 to 8, 8 to 12 mg of Compound (I). Thus for example in the above mentioned compositions, including unit doses, or treatments the Benzenesulfonate or a pharmaceutically acceptable solvate thereof is present in an amount providing 1mg of Compound (I); the Benzenesulfonate or a pharmaceutically acceptable solvate thereof is present in an amount providing 2mg of Compound (I); the Benzenesulfonate or a pharmaceutically acceptable solvate thereof is present in an amount providing 3mg of Compound (I); or the Benzenesulfonate or a pharmaceutically acceptable solvate thereof is present in an amount providing 4mg of Compound (I).

The other antidiabetic agents are suitably selected from biguanides, sulphonylureas and alpha glucosidase inhibitors. Suitable antidiabetic agents are those disclosed in WO98/57649, WO98/57634, WO98/57635, WO98/57636, WO99/03477, WO99/03476.

The contents of each of the above mentioned publications are incorporated herein by reference as if set forth herein.

No adverse toxicological effects are indicated in the above mentioned treatments for the compounds of the invention.

The following Examples illustrate the invention but do not limit it in any way.

### Reference Example 1 5-[4-[2-(N-Methyl-N-(2-pyridyl)amino)ethoxy]benzyl]thiazolidine-2,4-dione benzenesulfonate Form I

A mixture of tetrahydrofuran (THF, 30 mL) and 5-[4-[2-(N-Methyl-N-(2-pyridyl)amino)ethoxy]benzyl] thiazolidine-2,4-dione (3.0 g) was heated to 50°C. A solution of benzenesulfonic acid (1.33 g) in THF (10 mL) was added to the stirred solution and the mixture was heated to reflux until a white suspension was formed. The stirred mixture was cooled to 21°C over approximately 1 hour and the product collected by filtration and dried under vacuum over phosphorus pentoxide for 16 hours to give 5-[4-[2-(N-methyl-N-(2-pyridyl)amino)ethoxy]benzyl]thiazolidine-2,4-dione benzenesulfonate (4.4 g) as a white crystalline solid.

¹H-NMR (d6-DMSO): consistent with 5-[4-[2-(N-methyl-N-(2-pyridyl)amino)ethoxy]benzyl]thiazolidine-2,4-dione benzenesulfonate with a propan-2-ol content 0.8% wt/wt.

Water content (Karl Fischer): 0.8% wt/wt.

### Reference Example 2 5-[4-[2-(N-Methyl-N-(2-pyridyl)amino)ethoxy]benzyl]thiazolidine-2,4-dione benzenesulfonate Form I

A suspension of 5-[4-[2-(N-methyl-N-(2-pyridyl)amino)ethoxy]benzyl]thiazolidine-2,4-dione (3.0 g) in propan-2-ol (90 mL) was stirred and heated to reflux to form a clear solution. A solution of benzenesulfonic acid (1.33 g) in propan-2-ol (10 mL) was added to the solution which was heated at reflux until crystallisation was observed. The stirred mixture was then cooled to 21°C over approximately 1 hour. The product was collected by filtration, washed with 2-propanol (25 mL) and dried under vacuum over phosphorus pentoxide for 16 hours to give 5-[4-[2-(N-methyl-N-(2-pyridyl)amino)ethoxy]benzyl]thiazolidine-2,4-dione benzenesulfonate (3.6 g) as a white crystalline solid.

### Characterising data for the Benzenesulfonate Form I recorded for the product of Reference Example 1

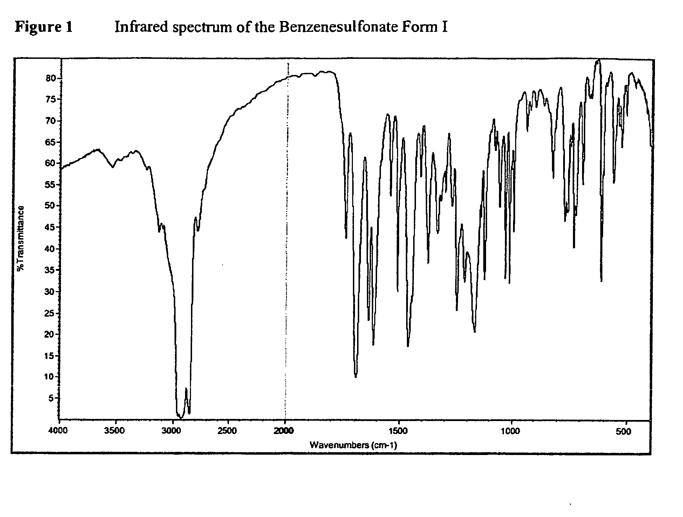

The infrared absorption spectrum of a mineral oil dispersion of the product was obtained using a Nicolet 710 FT-IR spectrometer at 2 cm⁻¹ resolution **(Figure 1**). Data were digitised at 1 cm⁻¹ intervals. Bands were observed at: 2786, 1742, 1696, 1643, 1620, 1546, 1512, 1411, 1334, 1269, 1247, 1214, 1168, 1124, 1058, 1033, 1016, 997, 935, 822, 766, 727, 689, 613, 561, 526, 506 cm⁻¹.

The infrared spectrum of the solid product was recorded using Perkin-Elmer Spectrum One FT-IR spectrometer fitted with a universal ATR accessory. Bands were observed at: 2980, 2783, 1742, 1694, 1643, 1620, 1546, 1513, 1446, 1411, 1388, 1333, 1270, 1246, 1214, 1166, 1124, 1058, 1033, 1016, 997, 935, 822, 754, 727, 717, 689, 657 cm⁻¹.

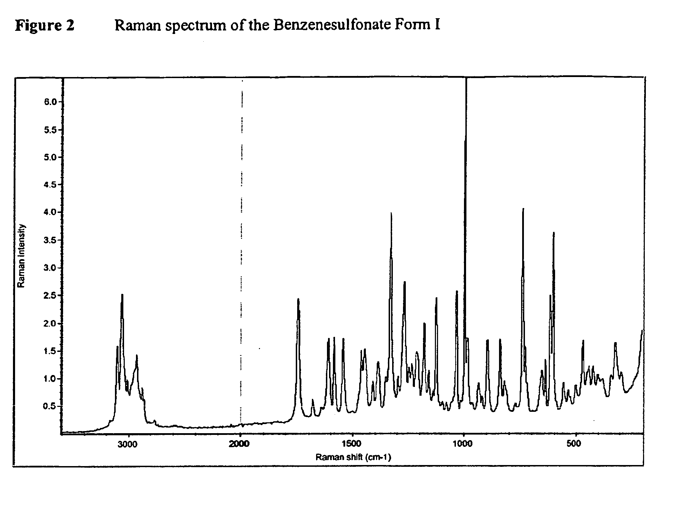

The Raman spectrum of the product (**Figure 2**) was recorded with the sample in an NMR tube using a Nicolet 960 E.S.P. FT-Raman spectrometer, at 4 cm⁻¹ resolution with excitation from a Nd:V04 laser (1064 nm) with a power output of 400mW. Bands were observed at: 3103, 3065, 2929, 1748, 1613, 1587, 1546, 1464, 1449, 1411, 1388, 1332, 1270, 1215, 1179, 1159, 1125, 1034, 997, 986, 935, 896, 840, 821, 741, 728, 617, 606, 471, 321 cm⁻¹.

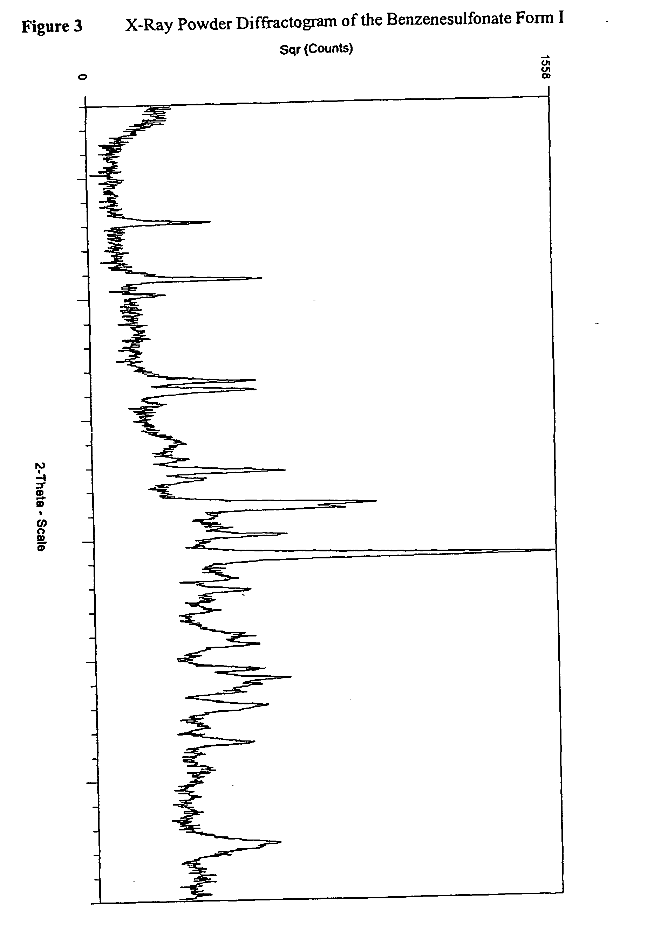

The X-Ray Powder Diffractogram pattern of the product (**Figure 3**) was recorded using the following acquisition conditions: Tube anode: Cu, Generator tension: 40 kV, Generator current: 40 mA, Start angle: 2.0 °2θ, End angle: 35.0 °2θ, Step size: 0.02 °2θ, Time per step: 2.5 seconds. Characteristic XRPD angles and relative intensities are recorded in Table 1.

**Table 1**

| **Angle** | **Rel. Intensity** |
|---|---|
| **2-Theta °** | **%** |
| 6.9 | 6.9 |
| 9.2 | 12.2 |
| 9.9 | 2.8 |
| 13.5 | 12.5 |
| 13.8 | 12.6 |
| 14.4 | 2.7 |
| 16.0 | 4 |
| 16.7 | 4.4 |
| 17.2 | 16.8 |
| 17.5 | 5.6 |
| 18.7 | 27.6 |
| 19.9 | 17.3 |
| 20.8 | 100 |
| 21.7 | 9.6 |
| 22.2 | 11.3 |
| 23.0 | 7.4 |
| 24.0 | 9.9 |
| 24.4 | 12.5 |
| 25.5 | 13.3 |
| 25.9 | 17.5 |
| 26.1 | 12.7 |
| 26.4 | 10.5 |
| 27.0 | 13.7 |
| 27.9 | 5.9 |
| 28.5 | 11.4 |
| 29.6 | 5.8 |
| 31.4 | 4.5 |
| 32.7 | 15.2 |

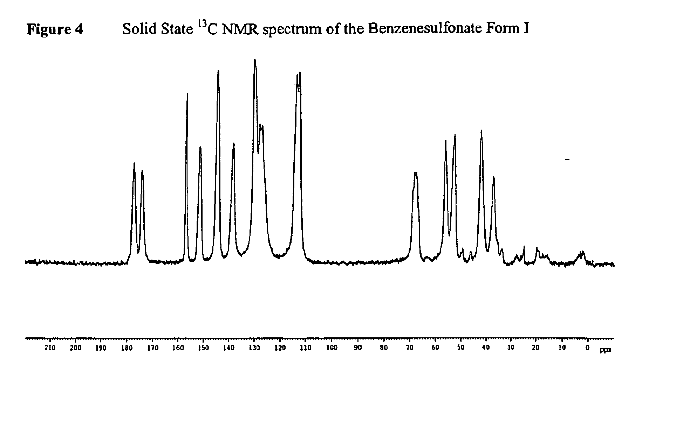

The solid-state NMR spectrum of the product (**Figure 4**) was recorded on a Bruker AMX360 instrument operating at 90.55 MHz: The solid was packed into a 4 mm zirconia MAS rotor fitted with a Kel-F cap and rotor spun at ca.10 kHz. The ¹³C MAS spectrum was acquired by cross-polarisation from Hartmann-Hahn matched protons (CP contact time 3 ms, repetition time 15 s) and protons were decoupled during acquisition using a two-pulse phase modulated (TPPM) composite sequence. Chemical shifts were externally referenced to the carboxylate signal of glycine at 176.4 ppm relative to TMS and were observed at: 37.3, 42.2, 52.8, 56.5, 67.6, 68.3, 112.8, 113.8, 127.3, 128.3, 130.5, 138.4, 144.7, 151.7, 156.9, 174.3, 177.5 ppm.

### Example 3: 5-[4-[2-(N-Methyl-N-(2-pyridyl)amino)ethoxy]benzyl]thiazolidine-2,4-dione benzenesulfonate Form II

A stirred suspension of 5-[4-[2-(N-methyl-N-(2-pyridyl)amino)ethoxy]benzyl]thiazolidine-2,4-dione (3.0 g) in industrial methylated spirit (IMS) (30 mL) was heated to reflux before a solution of benzenesulfonic acid (1.33 g) in IMS (10 mL) was added. The mixture was stirred at reflux for 5 minutes, at which point a clear solution was observed, and was then cooled to 21°C over 1 hour with stirring. The product was collected by filtration, washed with IMS (20 mL) and dried for 16 hours under vacuum to give 5-[4-[2-(N-methyl-N-(2-pyridyl)amino)ethoxy]benzyl]thiazolidine-2,4-dione benzenesulfonate (3.9 g) as a white crystalline solid.

### Example 4: 5-[4-[2-(N-Methyl-N-(2-pyridyl)amino)ethoxy]benzyl]thiazolidine-2,4-dione benzenesulfonate Form II

A solution of benzenesulfonic acid (1.33 g) in propan-2-ol (10 mL) was added to a stirred suspension of 5-[4-[2-(N-methyl-N-(2-pyridyl)amino)ethoxy]benzyl]thiazolidine-2,4-dione (3.0 g) in propan-2-ol (30 mL) at reflux. The mixture was maintained at reflux to give initially a clear solution followed by crystallisation. The stirred mixture was then cooled to 21°C over 40 minutes. The product was recovered by filtration and washed with propan-2-ol (10 ml) and dried under vacuum for 72 hours to give 5-[4-[2-(N-methyl-N-(2-pyridyl)amino)ethoxy]benzyl]thiazolidine-2,4-dione benzenesulfonate (4.1 g) as a white crystalline solid.

### Example 5: 5-[4-[2-(N-Methyl-N-(2-pyridyl)amino)ethoxy]benzyl]thiazolidine-2,4-dione benzenesulfonate Form II

A solution of benzenesulfonic acid (8.85 g) in propan-2-ol (65 mL) was added to a stirred suspension of 5-[4-[2-(N-methyl-N-(2-pyridyl)amino)ethoxy]benzyl]thiazolidine-2,4-dione (20.0 g) in propan-2-ol (200 mL) at reflux. The mixture was maintained at reflux for 5 minutes and then cooled to 21°C over approximately I hour. The solid was recovered by filtration, washed with propan-2-ol (50 mL) and dried at 50°C for 16 hours under vacuum to afford 5-[4-[2-(N-methyl-N-(2-pyridyl)amino)ethoxy]benzyl]thiazolidine-2,4-dione benzenesulfonate (27.17 g) as a white crystalline solid.

### Characterising data for the Benzenesulfonate Form II recorded for the product of Example 3

The infrared absorption spectrum of a mineral oil dispersion of the product was obtained using a Nicolet 710 FT-IR spectrometer at 2 cm⁻¹ resolution (**Figure 5**). Data were digitised at 1 cm⁻¹ intervals. Bands were observed at: 1751, 1703, 1646, 1617, 1584, 1549, 1512, 1463, 1415, 1331, 1304, 1237, 1176, 1123, 1097, 1081, 1068, 1034, 1016, 997, 926, 904, 837, 816, 772, 757, 740, 726, 713, 693, 666, 611, 565, 523 cm⁻¹.

The infrared spectrum of the solid product was recorded using Perkin-Elmer Spectrum One FT-IR spectrometer fitted with a universal ATR accessory. Bands were observed at: 3107, 3058, 2870, 1750, 1696, 1646, 1616, 1585, 1549, 1512, 1475, 1464, 1444, 1416, 1387, 1367, 1328, 1304, 1235, 1175, 1157, 1123, 1098, 1082, 1068, 1034, 1016, 996, 971, 925, 904, 837, 816, 771, 756, 741, 726, 712, 693, 666 cm⁻¹.

The Raman spectrum of the product (**Figure 6**) was recorded with the sample in an NMR tube using a Nicolet 960 E.S.P. FT-Raman spectrometer, at 4 cm⁻¹ resolution with excitation from a Nd:V04 laser (1064 nm) with a power output of 400mW. Bands were observed at: 3109, 3059, 2931, 2869, 1746, 1695, 1611, 1587, 1550, 1447, 1386, 1332, 1278, 1260, 1243, 1212, 1181, 1159, 1124, 1035, 997, 985, 904, 842, 741, 727, 664, 636, 604, 560, 508, 475, 435, 417, 320, 303 cm⁻¹.

The X-Ray Powder Diffractogram pattern of the product (**Figure 7**) was recorded using the following acquisition conditions: Tube anode: Cu, Generator tension: 40 kV, Generator current: 40 mA, Start angle: 2.0 °2θ, End angle: 35.0 °2θ, Step size: 0.02 °2θ, Time per step: 2.5 seconds. Characteristic XRPD angles and relative intensities are recorded in Table 2.

**Table 2**

| **Angle** | **Rel. Intensity** |
|---|---|
| **2-Theta °** | **%** |
| 6.3 | 3.9 |
| 8.1 | 56.2 |
| 10.3 | 26.7 |
| 12.3 | 9.1 |
| 13.3 | 7.1 |
| 13.7 | 3.6 |
| 14.2 | 13.2 |
| 14.8 | 22.5 |
| 15.1 | 24.6 |
| 16.4 | 52.7 |
| 17.5 | 9.3 |
| 18.5 | 100.0 |
| 19.1 | 35.2 |
| 19.4 | 5.0 |
| 19.7 | 5.2 |
| 20.5 | 6.0 |
| 20.8 | 12.1 |
| 21.2 | 10.4 |
| 21.4 | 9.8 |
| 21.7 | 51.8 |
| 22.7 | 26.3 |
| 22.9 | 35.4 |
| 23.4 | 21.6 |
| 23.6 | 28.4 |
| 24.6 | 25.6 |
| 25.3 | 34.4 |
| 25.6 | 7.0 |
| 26.1 | 11.1 |
| 26.6 | 13.9 |
| 27.0 | 12.2 |
| 27.5 | 18.1 |
| 27.8 | 16.0 |
| 28.6 | 5.8 |
| 29.0 | 3.6 |
| 29.9 | 9.2 |
| 30.6 | 9.5 |
| 31.4 | 24.4 |
| 32.1 | 8.1 |
| 33.2 | 7.7 |
| 33.7 | 12.3 |
| 34.7 | 5.7 |

The solid-state NMR spectrum of the product (**Figure 8**) was recorded on a Bruker AMX360 instrument operating at 90.55 MHz: The solid was packed into a 4 mm zirconia MAS rotor fitted with a Kel-F cap and rotor spun at ca.10 kHz. The ¹³C MAS spectrum was acquired by cross-polarisation from Hartmann-Hahn matched protons (CP contact time 3 ms, repetition time 15 s) and protons were decoupled during acquisition using a two-pulse phase modulated (TPPM) composite sequence. Chemical shifts were externally referenced to the carboxylate signal of glycine at 176.4 ppm relative to TMS and were observed at: 39.0, 41.0, 52.4, 55.0, 63.3, 65.1, 112.5, 113.4, 124.2, 127.8, 129.6, 131.6, 138.7, 141.3, 146.7, 150.5, 157.0, 171.7, 176.3 ppm.

### Properties of the Benzenesulfonate Form II, recorded for the product of Example 5

### Solubility of the Benzenesulfonate Form II

The solubility of the material was determined by adding water in aliquots from 1 to 1000ml to approximately 100mg of drug substance until the powder had dissolved. The visual solubility was confirmed by an HPLC assay of a saturated solution. Solubility: 1.4 mg/ml.

### Flow Properties of the Benzenesulfonate Form II

The ratio between the bulk density and the tapped bulk density (Hausner Ratio) of the Benzenesulfonate was determined using standard methods ("Pharmaceutics - The Science of Dosage Form Design", editor M. Aulton, 1988, published by:Churchill Livingstone). Hausner Ratio: 1.4

### Tₒₙₛₑₜ of the Benzenesulfonate Form II

The Tₒₙₛₑₜ of the drug substance was determined by Differential Scanning Calorimetry using a Perkin-Elmer DSC7 apparatus.
Tₒₙₛₑₜ (10°C/minute, closed pan): 180°C

### Melting Range of the Benzenesulfonate Form II

The melting range of the Benzenesulfonate Form II was determined according to the method described in the U.S. Pharmacopoeia, USP 23, 1995, <741> "Melting range or temperature, Procedure for Class Ia", using a Buchi 545 melting point instrument.
Melting range: 177.6-180.3°C

### Example 6 5-[4-[2-(N-Methyl-N-(2-pyridyl)amino)ethoxy]benzyl]thiazolidine-2,4-dione benzenesulfonate Form III

A solution of benzenesulfonic acid (4.43 g) in industrial methylated spirits (IMS) (20 mL) was added to a stirred suspension of 5-[4-[2-(N-methyl-N-(2-pyridyl)amino)ethoxy]benzyl]thiazolidine-2,4-dione (10.0 g) in IMS (100 mL) at reflux. The mixture was heated to reflux and was observed to give a clear solution after approximately 5 minutes. After cooling to 21 °C over approximately 1 hour the product was collected by filtration, washed with IMS (40 mL) and dried for 16 hours under vacuum to afford 5-[4-[2-(N-methyl-N-(2-pyridyl)amino)ethoxy]benzyl]thiazolidine-2,4-dione benzenesulfonate (12.54 g) as a white crystalline solid.

### Characterising data for the Benzenesulfonate Form III recorded for the product of Example 6

The infrared absorption spectrum of a mineral oil dispersion of the product was obtained using a Nicolet 710 FT-IR spectrometer at 2 cm⁻¹ resolution **(Figure 9).** Data were digitised at 1 cm⁻¹ intervals. Bands were observed at: 3118, 2770, 1750, 1697, 1640, 1614, 1586, 1545, 1511, 1462, 1418, 1332, 1313, 1292, 1272, 1235, 1210, 1163, 1123, 1094, 1078, 1067, 1033, 1014, 997, 923, 900, 874, 832, 817, 768, 728, 717, 706, 659, 619, 608, 568, 534, 519, 484 cm⁻¹.

The infrared spectrum of the solid product was recorded using Perkin-Elmer Spectrum One FT-IR spectrometer fitted with a universal ATR accessory. Bands were observed at: 3122, 2937, 2772, 1751, 1695, 1640, 1613, 1587, 1545, 1511, 1463, 1453, 1441, 1419, 1382, 1366, 1333, 1313, 1292, 1273, 1233, 1210, 1160, 1122, 1094, 1078, 1067, 1033, 1033, 1014, 997, 767, 728, 716, 706, 658 cm⁻¹.

The Raman spectrum of the product (**Figure 10**) was recorded with the sample in an NMR tube using a Nicolet 960 E.S.P. FT-Raman spectrometer, at 4 cm⁻¹ resolution with excitation from a Nd:V04 laser (1064 nm) with a power output of 400mW. Bands were observed at: 3102, 3076, 3060, 2920, 1751, 1612, 1585, 1544, 1442, 1419, 1382, 1313, 1294, 1239, 1207, 1182, 1171, 1144, 1123, 1097, 1033, 998, 974, 923, 901, 844, 825, 777, 739, 658, 637, 616, 604, 564, 519, 465, 416, 393, 368, 324, 308 cm⁻¹.

The X-Ray Powder Diffractogram pattern of the product (**Figure 11**) was recorded using the following acquisition conditions: Tube anode: Cu, Generator tension: 40 kV, Generator current: 40 mA, Start angle: 2.0 °2θ, End angle: 35.0 °2θ, Step size: 0.02 °2θ, Time per step: 2.5 seconds. Characteristic XRPD angles and relative intensities are recorded in Table 3.

**Table 3**

| **Angle** | **Rel. Intensity** |
|---|---|
| **2-Theta °** | **%** |
| 9.2 | 2.7 |
| 11.5 | 5.3 |
| 12.0 | 5.4 |
| 13.1 | 31.6 |
| 15.2 | 24.1 |
| 16.4 | 19.6 |
| 17.1 | 18.5 |
| 17.5 | 19.2 |
| 17.8 | 15.4 |
| 18.3 | 38.1 |
| 19.1 | 22.0 |
| 19.4 | 100.0 |
| 19.9 | 6.1 |
| 21.1 | 24.8 |
| 21.8 | 28.3 |
| 22.5 | 23.7 |
| 22.9 | 30.1 |
| 23.2 | 18.6 |
| 23.7 | 27.6 |
| 24.1 | 11.9 |
| 25.1 | 24.8 |
| 25.3 | 21.3 |
| 25.7 | 6.0 |
| 26.4 | 11.7 |
| 27.0 | 17.7 |
| 27.5 | 15.0 |
| 28.1 | 7.0 |
| 29.3 | 20.7 |
| 29.6 | 12.9 |
| 30.3 | 19.4 |
| 30.8 | 9.7 |
| 31.3 | 6.3 |
| 31.8 | 6.3 |
| 32.3 | 7.1 |
| 32.7 | 9.4 |
| 32.9 | 12.3 |
| 33.6 | 7.9 |

The solid-state NMR spectrum of the product (**Figure 12**) was recorded on a Bruker AMX360 instrument operating at 90.55 MHz: The solid was packed into a 4 mm zirconia MAS rotor fitted with a Kel-F cap and rotor spun at ca.10 kHz. The ¹³C MAS spectrum was acquired by cross-polarisation from Hartmann-Hahn matched protons (CP contact time 3 ms, repetition time 15 s) and protons were decoupled during acquisition using a two-pulse phase modulated (TPPM) composite sequence. Chemical shifts were externally referenced to the carboxylate signal of glycine at 176.4 ppm relative to TMS and were observed at: 36.0, 40.8, 48.5, 55.9, 64.4, 108.6, 112.1, 114.6, 118.3, 127.0, 127.7, 130.1, 131.4, 139.1, 142.8, 146.6, 150.2, 156.6, 171.6, 175.0, 176.3 ppm.

### Properties of the Benzenesulfonate Form III, recorded for the product of Example 6

### Solubility of the Benzenesulfonate Form III

The solubility of the material was determined by adding water in aliquots from 1 to 1000ml to approximately 100mg of drug substance until the powder had dissolved. The visual solubility was confirmed by an HPLC assay of a saturated solution. Solubility: 1.1 mg/ml.

### Flow Properties of the Benzenesulfonate Form III

The ratio between the bulk density and the tapped bulk density (Hausner Ratio) of the Benzenesulfonate was determined using standard methods ("Pharmaceutics - The Science of Dosage Form Design", editor M. Aulton, 1988, published by:Churchill Livingstone). Hausner Ratio: 1.2

### Tₒₙₛₑₜ of the Benzenesulfonate Form III

The Tₒₙₛₑₜ of the drug substance was determined by Differential Scanning Calorimetry using a Perkin-Elmer DSC7 apparatus.
Tₒₙₛₑₜ (10°C/minute, closed pan): 176°C

### Melting Range of the Benzenesulfonate Form III

The melting range of the Benzenesulfonate Form III was determined according to the method described in the U.S. Pharmacopoeia, USP 23, 1995, <741> "Melting range or temperature, Procedure for Class Ia", using a Buchi 545 melting point instrument.
Melting range: 176.9-177.3°C

### Example 7: 5-[4-[2-(N-Methyl-N-(2-pyridyl)amino)ethoxy]benzyl]thiazolidine-2,4-dione benzenesulfonate propan-2-ol solvate

A solution of benzenesulfonic acid (4.43 g) in propan-2-ol (20 mL) was added to a stirred suspension of 5-[4-[2-(N-methyl-N-(2-pyridyl)amino)ethoxy]benzyl]thiazolidine-2,4-dione (10.0 g) in propan-2-ol (100 mL) at reflux. The mixture was stirred at reflux for 10 minutes to give a clear solution. The stirred solution was cooled to 80°C and maintained at that temperature until crystallisation was observed. The mixture was then cooled to 21 °C over 60 minutes with stirring. The product was collected by filtration, washed with propan-2-ol (30 mL) and dried under vacuum at 21°C for 37 hours to give 5-[4-[2-(N-methyl-N-(2-pyridyl)amino)ethoxy]benzyl]thiazolidine-2,4-dione benzenesulfonate propan-2-ol solvate as a white crystalline solid (14.7 g).

¹H-NMR (d6-DMSO): consistent with 5-[4-[2-(N-methyl-N-(2-pyridyl)amino)ethoxy]benzyl]thiazolidine-2,4-dione benzenesulfonate with a propan-2-ol content 7.1% by weight.

### Water content (Karl Fischer): 1.6% wt/wt.

### Characterising data for the Benzenesulfonate propan-2-ol solvate recorded for the product of Example 7

The infrared absorption spectrum of a mineral oil dispersion of the product was obtained using a Nicolet 710 FT-IR spectrometer at 2 cm⁻¹ resolution **(Figure 13).** Data were digitised at 1 cm⁻¹ intervals. Bands were observed at: 3541, 1743, 1697, 1642, 1617, 1545, 1512, 1464, 1336, 1247, 1177, 1124, 1056, 1034, 1016, 997, 937, 823, 767, 755, 727, 690, 613, 559, 538, 506 cm⁻¹.

The infrared spectrum of the solid product was recorded using Perkin-Elmer Spectrum One FT-IR spectrometer fitted with a universal ATR accessory. Bands were observed at: 3545, 3130, 3021, 2784, 1743, 1696, 1642, 1616, 1545, 1513, 1465, 1444, 1412, 1385, 1334, 1267, 1246, 1177, 1123, 1056, 1033, 1015, 997, 938, 893, 823, 766, 755, 726, 719, 690 cm⁻¹.

The Raman spectrum of 5-[4-[2-(N-methyl-N-(2-pyridyl)amino)ethoxy]benzyl] thiazolidine-2,4-dione benzenesulfonate propan-2-ol solvate **(Figure 14)** was recorded with the sample in an NMR tube using a Nicolet 960 E.S.P. FT-Raman spectrometer, at 4 cm⁻¹ resolution with excitation from a Nd:V04 laser (1064 nm) with a power output of 400mW. Bands were observed at: 3107, 3061, 2970, 2928, 1750, 1612, 1586, 1545, 1464, 1449, 1412, 1392, 1331, 1267, 1248, 1234, 1214, 1179, 1124, 1034, 997, 938, 893, 840, 820, 742, 727, 638, 616, 607, 469, 440, 422, 317 cm⁻¹.

The X-Ray Powder Diffractogram pattern of the product **(Figure 15)** was recorded using the following acquisition conditions: Tube anode: Cu, Generator tension: 40 kV, Generator current: 40 mA, Start angle: 2.0 °2θ, End angle: 35.0 °2θ, Step size: 0.02 °2θ, Time per step: 2.5 seconds. Characteristic XRPD angles and relative intensities are recorded in Table 4.

**Table 4**

| **Angle** | **Rel. Intensity** |
|---|---|
| **2-Theta °** | **%** |
| 6.9 | 17.0 |
| 9.0 | 15.5 |
| 9.2 | 10.2 |
| 9.5 | 4.6 |
| 11.7 | 2.7 |
| 13.4 | 10.5 |
| 13.8 | 17.2 |
| 14.8 | 5.8 |
| 16.0 | 8.8 |
| 16.7 | 26.0 |
| 17.2 | 15.7 |
| 17.5 | 6.0 |
| 18.1 | 7.9 |
| 18.6 | 26.6 |
| 18.8 | 29.1 |
| 19.1 | 26.0 |
| 19.4 | 31.4 |
| 19.9 | 23.3 |
| 20.1 | 25.1 |
| 20.8 | 100.0 |
| 21.5 | 16.7 |
| 21.7 | 21.2 |
| 22.5 | 34.1 |
| 23.3 | 11.0 |
| 23.8 | 11.3 |
| 24.1 | 19.6 |
| 24.7 | 17.0 |
| 25.5 | 17.9 |
| 25.7 | 22.8 |
| 26.1 | 17.0 |
| 26.4 | 16.0 |
| 26.8 | 19.9 |
| 27.3 | 15.0 |
| 28.5 | 12.1 |
| 29.7 | 13.5 |
| 30.8 | 10.9 |
| 31.6 | 7.3 |
| 32.5 | 18.9 |
| 32.8 | 18.6 |
| 33.8 | 10.9 |

### Properties of the Benzenesulfonate propan-2-ol solvate, recorded for the product of Example 7

### Solubility of the Benzenesulfonate propan-2-ol solvate

The solubility of the material was determined by adding water in aliquots from 1 to 1000ml to approximately 100mg of drug substance until the powder had dissolved. The visual solubility was confirmed by an HPLC assay of a saturated solution. Solubility: 1.6 mg/ml.

### Flow Properties of the Benzenesulfonate propan-2-ol solvate

The ratio between the bulk density and the tapped bulk density (Hausner Ratio) of the Benzenesulfonate propan-2-ol solvate was determined using standard methods ("Pharmaceutics - The Science of Dosage Form Design", editor M. Aulton, 1988, published by:Churchill Livingstone).
Hausner Ratio: 1.5

### Tₒₙₛₑₜ of the Benzenesulfonate propan-2-ol solvate

The Tₒₙₛₑt of the drug substance was determined by Differential Scanning Calorimetry using a Perkin-Elmer DSC7 apparatus.
Tₒₙₛₑₜ (10°C/minute, closed pan): 172°C

### Melting Range of the Benzenesulfonate propan-2-ol solvate

The melting range of the Benzenesulfonate propan-2-ol solvate was determined according to the method described in the U.S. Pharmacopoeia, USP 23, 1995, <741> "Melting range or temperature, Procedure for Class Ia", using a Buchi 545 melting point instrument.
Melting range: 180.4-180.7°C

## Claims

1. A 5-[4-[2-(N-methyl-N-(2-pyridyl)amino)ethoxy]benzyl]thiazolidine-2,4-dione benzenesulfonate salt (Form II), **characterised in that** it provides:
(i) an infrared spectrum containing peaks at 1646, 837, 565 and 523 cm⁻¹; and/or
(ii) a Raman spectrum containing peaks at 1695, 1550 and 664 cm⁻¹;
(iii) an X-ray powder diffraction (XRPD) pattern containing peaks at 6.3, 8.1, 1.0.3, 12.3 and 14.2 degrees 2θ.

2. A 5-[4-[2-(N-methyl-N-(2-pyridyl)amino)ethoxy]benzyl]thiazolidine-2,4-dione benzenesulfonate salt, according to claim 1, **characterised in that** it provides an infrared spectrum in accordance with Figure 5.

3. A 5-[4-[2-(N-methyl-N-(2-pyridyl)amino)ethoxy]benzyl]thiazolidine-2,4-dione benzenesulfonate salt, according to claim 1 or 2, **characterised in that** it provides a Raman spectrum in accordance with Figure 6.

4. A 5-[4-[2-(N-methyl-N-(2-pyridyl)amina)ethoxy]benzyl]thiazolidine-2,4-dione benzenesulfonate salt, according to claim 1, 2 or 3, **characterised in that** it provides an X-Ray powder diffraction pattern (XRPD) with the characteristic XRPD angles and relative intensities:
| **Angle** | **Rel. Intensity** |
|---|---|
| **2-Theta °** | **%** |
| 6.3 | 3.9 |
| 8.1 | 56.2 |
| 10.3 | 26.7 |
| 12.3 | 9.1 |
| 13.3 | 7.1 |
| 13,7 | 3.6 |
| 14.2 | 13.2 |
| 14.8 | 22.5 |
| 15.1 | 24.6 |
| 16.4 | 52.7 |
| 17.5 | 9.3 |
| 18.5 | 100.0 |
| 19.1 | 35.2 |
| 19.4 | 5.0 |
| 19.7 | 5.2 |
| 20.5 | 6.0 |
| 20.8 | 12.1 |
| 21.2 | 10.4 |
| 21.4 | 9.8 |
| 21.7 | 51.8 |
| 22.7 | 26.3 |
| 22.9 | 35.4 |
| 23.4 | 21.6 |
| 23.6 | 28.4 |
| 24.6 | 25.6 |
| 25.3 | 34.4 |
| 25.6 | 7.0 |
| 26.1 | 11.1 |
| 26.6 | 13.9 |
| 27.0 | 12.2 |
| 27.5 | 18.1 |
| 27.8 | 16.0 |
| 28.6 | 5.8 |
| 29.0 | 3.6 |
| 29.9 | 9.2 |
| 30.6 | 9.5 |
| 31.4 | 24.4 |
| 32.1 | 8.1 |
| 33.2 | 7.7 |
| 33.7 | 12.3 |
| 34.7 | 5.7 |

5. A 5-[4-[2-(N-methyl-N-(2-pyridyl)amino)ethoxy]benzyl]thiazolidine-2,4-dione benzenesulfonate salt according to any one of claims 1 to 4, **characterised in that** it provides a Solid State ¹³C NMR spectrum in accordance with Figure 8.

6. A 5-[4-[2-(N-methyl-N-(2-pyridyl)amino)ethoxy]benzyl]thiazolidine-2,4-dione benzenesulfonate salt according to any one of claims 1 to 5, **characterised in that** it provides:
(i) an infrared spectrum in accordance with Figure 5; and
(ii) a Raman spectrum in accordance with Figure 6; and
(iii) an X-Ray powder diffraction pattern (XRPD) with the characteristic XRPD angles and relative intensities:
| **Angle** | **Rel. Intensity** |
|---|---|
| **2-Theta°** | **%** |
| 6.3 | 3.9 |
| 8.1 | 56.2 |
| 10.3 | 26.7 |
| 12.3 | 9.1 |
| 13.3 | 7.1 |
| 13.7 | 3.6 |
| 14.2 | 13.2 |
| 14.8 | 22.5 |
| 15.1 | 24.6 |
| 16.4 | 52.7 |
| 17.5 | 9.3 |
| 18.5 | 100.0 |
| 19.1 | 35.2 |
| 19.4 | 5.0 |
| 19.7 | 5.2 |
| 20.5 | 6.0 |
| 20.8 | 12.1 |
| 21.2 | 10.4 |
| 21.4 | 9.8 |
| 21.7 | 51.8 |
| 22.7 | 26.3 |
| 22.9 | 35.4 |
| 23.4 | 21.6 |
| 23.6 | 28.4 |
| 24.6 | 25.6 |
| 25.3 | 34.4 |
| 25.6 | 7.0 |
| 26.1 | 11.1 |
| 26.6 | 13.9 |
| 27.0 | 12.2 |
| 27.5 | 18.1 |
| 27.8 | 16.0 |
| 28.6 | 5.8 |
| 29.0 | 3.6 |
| 29.9 | 9.2 |
| 30.6 | 9.5 |
| 31.4 | 24.4 |
| 32.1 | 8.1 |
| 33.2 | 7.7 |
| 33.7 | 12.3 |
| 34.7 | 5.7 |
and
(iv) a Solid State ¹³C NMR spectrum in accordance with Figure 8.

7. A 5-[4-[2-(N-methyl-N-(2-pyridyl)amino)ethoxy]benzyl]thiazolidine-2,4-dione benzenesulfonate salt (Form III), **characterised in that** it provides:
(i) an infrared spectrum containing peaks at 1313, 1163, 706, 568 and 519 cm⁻¹; and/or
(ii) a Raman spectrum containing peaks at 2920,1442,1207,1144,658 and 308 cm⁻¹;
(iii) an X-ray powder diffraction (XRPD) pattern containing peaks at 11,5, 12.0, 13.1, 17.8 and 18.3 degrees 2θ.

8. A 5-[4-[2-(N-methyl-N-(2-pyridyl)amino)ethoxy]benzyl]thiazolidine-2,4-dione benzenesulfonate salt according to claim 7, **characterised in that** it provides an infrared spectrum in accordance with Figure 9.

9. A 5-[4-[2-(N-methyl-N-(2-pyridyl)amino)ethoxy]benzyl]thiazolidine-2,4-dione benzenesulfonate salt according to claim 7 or 8, **characterised in that** it provides a Raman spectrum in accordance with Figure 10.

10. A 5-[4-[2-(N-methyl-N-(2-pyridyl)amino)ethoxy]benzyl]thiazolidine-2,4-dione benzenesulfonate salt according to claim 7, 8 or 9 , **characterised in that** it provides an X-Ray powder diffraction pattern (XRPD) with the characteristic XRPD angles and relative intensities:
| **Angle** | **Rel. Intensity** |
|---|---|
| **2-Theta °** | **%** |
| 9.2 | 2.7 |
| 11.5 | 5.3 |
| 12.0 | 5.4 |
| 13.1 | 31.6 |
| 15.2 | 24.1 |
| 16.4 | 19.6 |
| 17.1 | 18.5 |
| 17.5 | 19.2 |
| 17.8 | 15.4 |
| 18.3 | 38.1 |
| 19.1 | 22.0 |
| 19.4 | 100.0 |
| 19.9 | 6.1 |
| 21.1 | 24.8 |
| 21.8 | 28.3 |
| 22.5 | 23.7 |
| 22.9 | 30.1 |
| 23.2 | 18.6 |
| 23.7 | 27.6 |
| 24.1 | 11.9 |
| 25.1 | 24.8 |
| 25.3 | 21.3 |
| 25.7 | 6.0 |
| 26.4 | 11.7 |
| 27.0 | 17.7 |
| 27.5 | 15.0 |
| 28.1 | 7.0 |
| 29.3 | 20.7 |
| 29.6 | 12.9 |
| 30.3 | 19.4 |
| 30.8 | 9.7 |
| 31.3 | 6.3 |
| 31.8 | 6.3 |
| 32.3 | 7.1 |
| 32.7 | 9.4 |
| 32.9 | 12.3 |
| 33.6 | 7.9 |

11. A 5-[4-[2-(N-methyl-N-(2-pyridyl)amino)ethoxy]benzyl]thiazolidine-2,4-dione benzenesulfonate salt according to any one of claims 7 to 10, **characterised in that** it provides a Solid State ¹³C NMR spectrum in accordance with Figure 12.

12. A 5-[4-[2-(N-methyl-N-(2-pyridyl)amino)ethoxy]benzyl]thiazolidine-2,4-dione benzenesulfonate salt according to any one of claims 7 to 11, **characterised in that** it provides
(i) an infrared spectrum in accordance with Figure 9; and
(ii) a Raman spectrum in accordance with Figure 10; and
(iii) an X-Ray powder diffraction pattern (XRPD) with the characteristic XRPD angles and relative intensities:
| **Angle** | **Rel. Intensity** |
|---|---|
| **2-Theta°** | **%** |
| 9.2 | 2.7 |
| 11.5 | 5.3 |
| 12.0 | 5.4 |
| 13.1 | 31.6 |
| 15.2 | 24.1 |
| 16.4 | 19.6 |
| 17.1 | 18.5 |
| 17.5 | 19.2 |
| 17.8 | 15.4 |
| 18.3 | 38.1 |
| 19.1 | 22.0 |
| 19.4 | 100.0 |
| 19.9 | 6.1 |
| 21.1 | 24.8 |
| 21.8 | 28.3 |
| 22.5 | 23.7 |
| 22.9 | 30.1 |
| 23.2 | 18.6 |
| 23.7 | 27.6 |
| 24.1 | 11.9 |
| 25.1 | 24.8 |
| 25.3 | 21.3 |
| 25.7 | 6.0 |
| 26.4 | 11.7 |
| 27.0 | 17.7 |
| 27.5 | 15.0 |
| 28.1 | 7.0 |
| 29.3 | 20.7 |
| 29.6 | 12.9 |
| 30.3 | 19.4 |
| 30.8 | 9.7 |
| 31.3 | 6.3 |
| 31.8 | 6.3 |
| 32.3 | 7.1 |
| 32.7 | 9.4 |
| 32.9 | 12.3 |
| 33.6 | 7.9 |
and
(iv) a Solid State ¹³C NMR spectrum in accordance with Figure 12.

13. A 5-[4-[2-(N-methyl-N-(2-pyridyl)amino)ethoxy]benzyl]thiazolidine-2,4-dione benzenesulfonate salt propan-2-ol solvate, **characterised in that** it provides:
(i) an infrared spectrum containing a peak at 3541 cm⁻¹; and/or
(ii) a Raman spectrum containing peaks at 893 and 317 cm⁻¹;
(iii) an X-ray powder diffraction (XRPD) pattern containing peaks at 9.5, 13.3, 20.1 and 26.8 degrees 2θ.

14. A 5-[4-[2-(N-methyl-N-(2-pyridyl)amino)ethoxy]benzyl]thiazolidine-2,4-dione benzenesulfonate salt according to claim 13, **characterised in that** it provides an infrared spectrum in accordance with Figure 13.

15. A 5-[4-[2-(N-methyl-N-(2-pyridyl)amino)ethoxy]benzyl]thiazolidine-2,4-dione benzenesulfonate salt according to claim 13 or 14, **characterised in that** it provides a Raman spectrum in accordance with Figure 14.

16. A 5-[4-[2-(N-methyl-N-(2-pyridyl)amino)ethoxy]benzyl]thiazolidine-2,4-dione benzenesulfonate salt according to claim 13, 14 or 15, **characterised in that** it provides an X-Ray powder diffraction pattern (XRPD) with the characteristic XRPD angles and relative intensities:
| **Angle** | **Rel. Intensity** | |
|---|---|---|
| **2-Theta °** | **%** | |
| 6.9 | 17.0 | |
| 9.0 | 15.5 | |
| 9.2 | 10.2 | |
| 9.5 | 4.6 | |
| 11.7 | 2.7 | |
| 13.4 | 10.5 | |
| 13.8 | 17.2 | |
| 14.8 | 5.8 | |
| 16.0 | 8.8 | |
| 16.7 | 26.0 | |
| 17.2 | 15.7 | |
| 17.5 | 6.0 | |
| 18.1 | 7.9 | |
| 18.6 | 26.6 | |
| 18.8 | 29.1 | |
| 19.1 | 26.0 | |
| 19.4 | 31.4 | |
| 19.9 | 23.3 | |
| 20.1 | 25.1 | |
| 20.8 | 100.0 | |
| 21.5 | 16.7 | |
| 21.7 | 21.2 | |
| 22.5 | 34.1 | |
| 23.3 | 11.0 | |
| 23.8 | 11.3 | |
| 24.1 | 19.6 | |
| 24.7 | 17.0 | |
| 25.5 | 17.9 | |
| 25.7 | 22.8 | |
| 26.1 | 17.0 | |
| 26.4 | 16.0 | |
| 26.8 | 19.9 | |
| 27.3 | 15.0 | |
| 28.5 | 12.1 | |
| 29.7 | 13.5 | |
| 30.8 | 10.9 | |
| 31.6 | 7.3 | |
| 32.5 | 18.9 | |
| 32.8 | 18.6 | |
| 33.8 | 10.9 | |

17. A 5-[4-[2-(N-methyl-N-(2-pyridyl)amino)ethoxy]benzyl]thiazolidine-2,4-dione benzenesulfonate salt according to any one of claims 13 to 16, **characterised in that** it provides:
(i) an infrared spectrum in accordance with Figure 13; and
(ii) a Raman spectrum in accordance with Figure 14; and
(iii) an X-Ray powder diffraction pattern (XRPD) with the characteristic XRPD angles and relative intensities:
| **Angle** | **Rel. Intensity** |
|---|---|
| **2-Theta °** | **%** |
| 6.9 | 17.0 |
| 9.0 | 15.5 |
| 9.2 | 10.2 |
| 9.5 | 4.6 |
| 11.7 | 2.7 |
| 13.4 | 10.5 |
| 13.8 | 17.2 |
| 14.8 | 5.8 |
| 16.0 | 8.8 |
| 16.7 | 26.0 |
| 17.2 | 15.7 |
| 17.5 | 6.0 |
| 18.1 | 7.9 |
| 18.6 | 26.6 |
| 18.8 | 29.1 |
| 19.1 | 26.0 |
| 19.4 | 31.4 |
| 19.9 | 23.3 |
| 20.1 | 25.1 |
| 20.8 | 100.0 |
| 21.5 | 16.7 |
| 21.7 | 21.2 |
| 22.5 | 34.1 |
| 23.3 | 11.0 |
| 23.8 | 11.3 |
| 24.1 | 19.6 |
| 24.7 | 17.0 |
| 25.5 | 17.9 |
| 25.7 | 22.8 |
| 26.1 | 17.0 |
| 26.4 | 16.0 |
| 26.8 | 19.9 |
| 27.3 | 15.0 |
| 28.5 | 12.1 |
| 29.7 | 13.5 |
| 30.8 | 10.9 |
| 31.6 | 7.3 |
| 32.5 | 18.9 |
| 32.8 | 18.6 |
| 33.8 | 10.9 |

18. A compound according to any one of claims 1 to 17, in isolated form.

19. A compound according to any one of claims 1 to 17, in crystalline form.

20. A process for preparing a compound according to any one of claims 1 to 19, **characterised in that** 5-[4-[2-(N-methyl-N-(2-pyridyl)amino)ethoxy]benzyl]thiazolidine-2,4-dione (Compound (I))or a salt thereof, preferably dispersed or dissolved in a suitable solvent, is reacted with a source of benzenesulfonate ion and the benzenesulfonate salt is recovered.

21. 5-[4-[2-(N-methyl-N-(2-pyridyl)amino)ethoxy]benzyl]thiazolidine-2,4-dione benzenesulfonate salt Form II or Form III or propan-2-ol solvate according to claim 1, 7 or 13, respectively, for use as an active therapeutic substance.

22. 5-[4-[2-(N-methyl-N-(2-pyridyl)amino)ethoxy]benzyl]thiazolidine-2,4-dione benzenesulfonate Form II or Form III or propan-2-ol solvate according to claim 1, 7 or 13, respectively, for use in the treatment and/or prophylaxis of diabetes mellitus, conditions associated with diabetes mellitus and certain complications thereof.

23. A use of 5-[4-[2-(N-methyl-N-(2-pyridyl)amino)ethoxy]benzyl]thiazolidine-2,4-dione benzenesulfonate Form II or Form III or propan-2-ol solvate according to claim 1, 7 or 13, respectively, for the manufacture of a medicament for the treatment and/or prophylaxis of diabetes mellitus, conditions associated with diabetes mellitus and certain complications thereof.

## Patentansprüche

1. 5-[4-[2-(N-Methyl-N-(2-pyridyl)amino)ethoxy]benzyl]thiazolidin-2,4-dion-benzolsulfonatSalz (Form II), **dadurch gekennzeichnet, dass** es
(i) ein Infrarotspektrum mit Banden bei 1646, 837, 565 und 523 cm⁻¹; und/oder
(ii) ein Ramanspektrum mit Banden bei 1695, 1550 und 664 cm⁻¹;
(iii) ein Röntgenpulver-Beugungsmuster (XRPD) mit Banden bei 6,3, 8,1, 10,3, 12,3 und 14,2 Grad 2θ,
aufweist.

2. 5-[4-[2-(N-Methyl-N-(2-pyridyl)amino)ethoxy]benzyl]thiazolidin-2,4-dion-benzolsulfonatSalz gemäß Anspruch 1, **dadurch gekennzeichnet, dass** es ein Infrarotspektrum gemäß Figur 5 aufweist.

3. 5-[4-[2-(N-Methyl-N-(2-pyridyl)amino)ethoxy]benzyl]thiazolidin-2,4-dion-benzolsulfonatSalz gemäß Anspruch 1 oder 2, **dadurch gekennzeichnet, dass** es ein Ramanspektrum gemäß Figur 6 aufweist.

4. 5-[4-[2-(N-Methyl-N-(2-pyridyl)amino)ethoxy]benzyl]thiazolidin-2,4-dion-benzolsulfonatSalz gemäß Anspruch 1, 2 oder 3, **dadurch gekennzeichnet, dass** es ein Röntgenpulver-Beugungsmuster (XRPD) mit folgenden charakteristischen XRPD-Winkeln und relativen Intensitäten:
| **Winkel** | **Rel. Intensität** |
|---|---|
| **2-Theta** ° | **%** |
| 6,3 | 3,9 |
| 8,1 | 56,2 |
| 10,3 | 26,7 |
| 12,3 | 9,1 |
| 13,3 | 7,1 |
| 13,7 | 3,6 |
| 14,2 | 13,2 |
| 14,8 | 22,5 |
| 15,1 | 24,6 |
| 16,4 | 52,7 |
| 17,5 | 9,3 |
| 18,5 | 100,0 |
| 19,1 | 35,2 |
| 19,4 | 5,0 |
| 19,7 | 5,2 |
| 20,5 | 6,0 |
| 20,8 | 12,1 |
| 21,2 | 10,4 |
| 21,4 | 9,8 |
| 21,7 | 51,8 |
| 22,7 | 26,3 |
| 22,9 | 35,4 |
| 23,4 | 21,6 |
| 23,6 | 28,4 |
| 24,6 | 25,6 |
| 25,3 | 34,4 |
| 25,6 | 7,0 |
| 26,1 | 11,1 |
| 26,6 | 13,9 |
| 27,0 | 12,2 |
| 27,5 | 18,1 |
| 27,8 | 16,0 |
| 28,6 | 5,8 |
| 29,0 | 3,6 |
| 29,9 | 9,2 |
| 30,6 | 9,5 |
| 31,4 | 24,4 |
| 32,1 | 8,1 |
| 33,2 | 7,7 |
| 33,7 | 12,3 |
| 34,7 | 5,7 |
aufweist.

5. 5-[4-[2-(N-Methyl-N-(2-pyridyl)amino)ethoxy]benzyl]thiazolidin-2,4-dion-benzolsulfonatSalz gemäß einem der Ansprüche 1 bis 4, **dadurch gekennzeichnet, dass** es ein Festkörper-¹³C-NMR-Spektrum gemäß Figur 8 aufweist.

6. 5-[4-[2-(N-Methyl-N-(2-pyridyl)amino)ethoxy]benzyl]thiazolidin-2,4-dion-benzolsulfonatSalz gemäß einem der Ansprüche 1 bis 5, **dadurch gekennzeichnet, dass** es
(i) ein Infrarotspektrum gemäß Figur 5; und
(ii) ein Ramanspektrum gemäß Figur 6; und
(iii) ein Röntgenpulver-Beugungsmuster (XRPD) mit folgenden charakteristischen XRPD-Winkeln und relativen Intensitäten:
| **Winkel** | **Rel. Intensität** |
|---|---|
| **2-Theta °** | **%** |
| 6,3 | 3,9 |
| 8,1 | 56,2 |
| 10,3 | 26,7 |
| 12,3 | 9,1 |
| 13,3 | 7,1 |
| 13,7 | 3,6 |
| 14,2 | 13,2 |
| 14,8 | 22,5 |
| 15,1 | 24,6 |
| 16,4 | 52,7 |
| 17,5 | 9,3 |
| 18,5 | 100,0 |
| 19,1 | 35,2 |
| 19,4 | 5,0 |
| 19,7 | 5,2 |
| 20,5 | 6,0 |
| 20,8 | 12,1 |
| 21,2 | 10,4 |
| 21,4 | 9,8 |
| 21,7 | 51,8 |
| 22,7 | 26,3 |
| 22,9 | 35,4 |
| 23,4 | 21,6 |
| 23,6 | 28,4 |
| 24,6 | 25,6 |
| 25,3 | 34,4 |
| 25,6 | 7,0 |
| 26,1 | 11,1 |
| 26,6 | 13,9 |
| 27,0 | 12,2 |
| 27,5 | 18,1 |
| 27,8 | 16,0 |
| 28,6 | 5,8 |
| 29,0 | 3,6 |
| 29,9 | 9,2 |
| 30,6 | 9,5 |
| 31,4 | 24,4 |
| 32,1 | 8,1 |
| 33,2 | 7,7 |
| 33,7 | 12,3 |
| 34,7 | 5,7 |
und
(iv) ein Festkörper-¹³C-NMR-Spektrum gemäß Figur 8 aufweist.

7. 5-[4-[2-(N-Methyl-N-(2-pyridyl)amino)ethoxy]benzyl]thiazolidin-2,4-dion-benzolsulfonatSalz (Form III), **dadurch gekennzeichnet, dass** es
(i) ein Infrarotspektrum mit Banden bei 1313, 1163, 706, 568 und 519 cm⁻¹; und/oder
(ii) ein Ramanspektrum mit Banden bei 2920, 1442, 1207, 1144, 658 und 308 cm⁻¹;
(iii) ein Röntgenpulver-Beugungsmuster (XRPD) mit Banden bei 11,5, 12,0, 13,1, 17,8 und 18,3 Grad 2θ
aufweist.

8. 5-[4-[2-(N-Methyl-N-(2-pyridyl)amino)ethoxy]benzyl]thiazolidin-2,4-dion-benzolsulfonatSalz gemäß Anspruch 7, **dadurch gekennzeichnet, dass** es ein Infrarotspektrum gemäß Figur 9 aufweist.

9. 5-[4-[2-(N-Methyl-N-(2-pyridyl)amino)ethoxy]benzyl]thiazolidin-2,4-dion-benzolsulfonatSalz gemäß Anspruch 7 oder 8, **dadurch gekennzeichnet, dass** es ein Ramanspektrum gemäß Figur 10 aufweist.

10. 5-[4-[2-(N-Methyl-N-(2-pyridyl)amino)ethoxy]benzyl]thiazolidin-2,4-dion-benzolsulfonatSalz gemäß Anspruch 7, 8 oder 9, **dadurch gekennzeichnet, dass** es ein Röntgenpulver-Beugungsmuster (XRPD) mit folgenden charakteristischen XRPD-Winkeln und relativen Intensitäten:
| **Winkel** | **Rel. Intensität** |
|---|---|
| **2-Theta °** | **%** |
| 9,2 | 2,7 |
| 11,5 | 5,3 |
| 12,0 | 5,4 |
| 13,1 | 31,6 |
| 15,2 | 24,1 |
| 16,4 | 19,6 |
| 17,1 | 18,5 |
| 17,5 | 19,2 |
| 17,8 | 15,4 |
| 18,3 | 38,1 |
| 19,1 | 22,0 |
| 19,4 | 100,0 |
| 19,9 | 6,1 |
| 21,1 | 24,8 |
| 21,8 | 28,3 |
| 22,5 | 23,7 |
| 22,9 | 30,1 |
| 23,2 | 18,6 |
| 23,7 | 27,6 |
| 24,1 | 11,9 |
| 25,1 | 24,8 |
| 25,3 | 21,3 |
| 25,7 | 6,0 |
| 26,4 | 11,7 |
| 27,0 | 17,7 |
| 27,5 | 15,0 |
| 28,1 | 7,0 |
| 29,3 | 20,7 |
| 29,6 | 12,9 |
| 30,3 | 19,4 |
| 30,8 | 9,7 |
| 31,3 | 6,3 |
| 31,8 | 6,3 |
| 32,3 | 7,1 |
| 32,7 | 9,4 |
| 32,9 | 12,3 |
| 33,6 | 7,9 |
aufweist.

11. 5-[4-[2-(N-Methyl-N-(2-pyridyl)amino)ethoxy]benzyl]thiazolidin-2,4-dion-benzolsulfonatSalz gemäß einem der Ansprüche 7 bis 10, **dadurch gekennzeichnet, dass** es ein Festkörper-¹³C-NMR-Spektrum gemäß Figur 12 aufweist.

12. 5-[4-[2-(N-Methyl-N-(2-pyridyl)amino)ethoxy]benzyl]thiazolidin-2,4-dion-benzolsulfonatSalz gemäß einem der Ansprüche 7 bis 11, **dadurch gekennzeichnet, dass** es
(i) ein Infrarotspektrum gemäß mit Figur 9; und
(ii) ein Ramanspektrum gemäß Figur 10; und
(iii) ein Röntgenpulver-Beugungsmuster (XRPD) mit folgenden charakteristischen XRPD-Winkeln und relativen Intensitäten:
| **Winkel** | **Rel. Intensität** |
|---|---|
| **2-Theta °** | **%** |
| 9,2 | 2,7 |
| 11,5 | 5,3 |
| 12,0 | 5,4 |
| 13,1 | 31,6 |
| 15,2 | 24,1 |
| 16,4 | 19,6 |
| 17,1 | 18,5 |
| 17,5 | 19,2 |
| 17,8 | 15,4 |
| 18,3 | 38,1 |
| 19,1 | 22,0 |
| 19,4 | 100,0 |
| 19,9 | 6,1 |
| 21,1 | 24,8 |
| 21,8 | 28,3 |
| 22,5 | 23,7 |
| 22,9 | 30,1 |
| 23,2 | 18,6 |
| 23,7 | 27,6 |
| 24,1 | 11,9 |
| 25,1 | 24,8 |
| 25,3 | 21,3 |
| 25,7 | 6,0 |
| 26,4 | 11,7 |
| 27,0 | 17,7 |
| 27,5 | 15,0 |
| 28,1 | 7,0 |
| 29,3 | 20,7 |
| 29,6 | 12,9 |
| 30,3 | 19,4 |
| 30,8 | 9,7 |
| 31,3 | 6,3 |
| 31,8 | 6,3 |
| 32,3 | 7,1 |
| 32,7 | 9,4 |
| 32,9 | 12,3 |
| 33,6 | 7,9 |
und
(iv) ein Festkörper-¹³C-NMR-Spektrum gemäß Figur 12 aufweist.

13. Propan-2-ol-solvat des 5-[4-[2-(N-Methyl-N-(2-pyridyl)amino)ethoxy]benzyl]thiazolidin-2,4-dion-benzolsulfonat-Salzes, **dadurch gekennzeichnet, dass** es
(i) ein Infrarotspektrum mit einer Bande bei 3541 cm⁻¹; und/oder
(ii) ein Ramanspektrum mit Banden bei 893 und 317 cm⁻¹;
(iii) ein Röntgenpulver-Beugungsmuster (XRPD) mit Banden bei 9,5, 13,3, 20,1 und 26,8 Grad 2θ
aufweist.

14. 5-[4-[2-(N-Methyl-N-(2-pyridyl)amino)ethoxy]benzyl]thiazolidin-2,4-dion-benzolsulfonatSalz gemäß Anspruch 13, **dadurch gekennzeichnet, dass** es ein Infrarotspektrum gemäß Figur 13 aufweist.

15. 5-[4-[2-(N-Methyl-N-(2-pyridyl)amino)ethoxy]benzyl]thiazolidin-2,4-dion-benzolsulfonatSalz gemäß Anspruch 13 oder 14, **dadurch gekennzeichnet, dass** es ein Ramanspektrum gemäß Figur 14 aufweist.

16. 5-[4-[2-(N-Methyl-N-(2-pyridyl)amino)ethoxy]benzyl]thiazolidin-2,4-dion-benzolsulfonatSalz gemäß Anspruch 13, 14 oder 15, **dadurch gekennzeichnet, dass** es ein Röntgenpulver-Beugungsmuster (XRPD) mit folgenden charakteristischen XRPD-Winkeln und relativen Intensitäten:
| **Winkel** | **Rel. Intensität** |
|---|---|
| **2-Theta °** | **%** |
| 6,9 | 17,0 |
| 9,0 | 15,5 |
| 9,2 | 10,2 |
| 9,5 | 4,6 |
| 11,7 | 2,7 |
| 13,4 | 10,5 |
| 13,8 | 17,2 |
| 14,8 | 5,8 |
| 16,0 | 8,8 |
| 16,7 | 26,0 |
| 17,2 | 15,7 |
| 17,5 | 6,0 |
| 18,1 | 7,9 |
| 18,6 | 26,6 |
| 18,8 | 29,1 |
| 19,1 | 26,0 |
| 19,4 | 31,4 |
| 19,9 | 23,3 |
| 20,1 | 25,1 |
| 20,8 | 100,0 |
| 21,5 | 16,7 |
| 21,7 | 21,2 |
| 22,5 | 34,1 |
| 23,3 | 11,0 |
| 23,8 | 11,3 |
| 24,1 | 19,6 |
| 24,7 | 17,0 |
| 25,5 | 17,9 |
| 25,7 | 22,8 |
| 26,1 | 17,0 |
| 26,4 | 16,0 |
| 26,8 | 19,9 |
| 27,3 | 15,0 |
| 28,5 | 12,1 |
| 29,7 | 13,5 |
| 30,8 | 10,9 |
| 31,6 | 7,3 |
| 32,5 | 18,9 |
| 32,8 | 18,6 |
| 33,8 | 10,9 |
aufweist.

17. 5-[4-[2-(N-Methyl-N-(2-pyridyl)amino)ethoxy]benzyl]thiazolidin-2,4-dion-benzolsulfonatSalz gemäß einem der Ansprüche 13 bis 16, **dadurch gekennzeichnet, dass** es
(i) ein Infrarotspektrum gemäß Figur 13; und
(ii) ein Ramanspektrum gemäß Figur 14; und
(iii)ein Röntgenpulver-Beugungsmuster (XRPD) mit folgenden charakteristischen XRPD-Winkeln und relativen Intensitäten:
| **Winkel** | **Rel. Intensität** |
|---|---|
| **2-Theta °** | **%** |
| 6,9 | 17,0 |
| 9,0 | 15,5 |
| 9,2 | 10,2 |
| 9,5 | 4,6 |
| 11,7 | 2,7 |
| 13,4 | 10,5 |
| 13,8 | 17,2 |
| 14,8 | 5,8 |
| 16,0 | 8,8 |
| 16,7 | 26,0 |
| 17,2 | 15,7 |
| 17,5 | 6,0 |
| 18,1 | 7,9 |
| 18,6 | 26,6 |
| 18,8 | 29,1 |
| 19,1 | 26,0 |
| 19,4 | 31,4 |
| 19,9 | 23,3 |
| 20,1 | 25,1 |
| 20,8 | 100,0 |
| 21,5 | 16,7 |
| 21,7 | 21,2 |
| 22,5 | 34,1 |
| 23,3 | 11,0 |
| 23,8 | 11,3 |
| 24,1 | 19,6 |
| 24,7 | 17,0 |
| 25,5 | 17,9 |
| 25,7 | 22,8 |
| 26,1 | 17,0 |
| 26,4 | 16,0 |
| 26,8 | 19,9 |
| 27,3 | 15,0 |
| 28,5 | 12,1 |
| 29,7 | 13,5 |
| 30,8 | 10,9 |
| 31,6 | 7,3 |
| 32,5 | 18,9 |
| 32,8 | 18,6 |
| 33,8 | 10,9 |
aufweist.

18. Verbindung gemäß einem der Ansprüche 1 bis 17 in isolierter Form.

19. Verbindung gemäß einem der Ansprüche 1 bis 17 in kristalliner Form.

20. Verfahren zur Herstellung einer Verbindung gemäß einem der Ansprüche 1 bis 19, **dadurch gekennzeichnet, dass** 5-[4-[2-(N-Methyl-N-(2-pyridyl)amino)ethoxy]benzyl]thiazolidin-2,4-dion (Verbindung (I)) oder ein Salz davon, bevorzugt dispergiert oder gelöst in einem geeigneten Lösungsmittel, mit einer Benzolsulfonationenquelle umgesetzt und das Benzolsulfonatsalz isoliert wird.

21. 5-[4-[2-(N-Methyl-N-(2-pyridyl)amino)ethoxy]benzyl]thiazolidin-2,4-dion-benzolsulfonatSalz Form II oder Form III oder das Propan-2-ol-solvat gemäß Anspruch 1, 7 bzw. 13, zur Verwendung als therapeutischer Wirkstoff.

22. 5-[4-[2-(N-Methyl-N-(2-pyridyl)amino)ethoxy]benzyl]thiazolidin-2,4-dion-benzolsulfonat Form II oder Form III oder das Propan-2-ol-solvat gemäß Anspruch 1, 7 bzw. 13, zur Verwendung in der Behandlung und/oder Vorbeugung von Diabetes Mellitus, mit Diabetes Mellitus verbundenen Erkrankungen und bestimmten Komplikationen hiervon.

23. Verwendung von 5-[4-[2-(N-Methyl-N-(2-pyridyl)amino)ethoxy]benzyl]thiazolidin-2,4-dion-benzolsulfonat Form II oder Form III oder dem Propan-2-ol-solvat gemäß Anspruch 1, 7 bzw. 13, zur Herstellung eines Medikamentes zur Behandlung und/oder Vorbeugung von Diabetes Mellitus, mit Diabetes Mellitus verbundenen Erkrankungen und bestimmten Komplikationen hiervon.

## Revendications

1. Benzènesulfonate de 5-[4-[2-(N-méthyl-N-(2-pyridyl)-amino)éthoxy]benzyl]thiazolidine-2,4-dione (Forme II), **caractérisé en ce qu'**il présente :
(i) un spectre infrarouge contenant des pics à 1646, 837, 565 et 523 cm⁻¹ ; et/ou
(ii) un spectre Raman contenant des pics à 1695, 1550 et 664 cm⁻¹ ;
(iii) un diagramme de diffraction des rayons X sur poudre (XRPD) contenant des pics à 6,3, 8,1, 10,3, 12,3 et 14,2 degrés 2θ.

2. Benzènesulfonate de 5-[4-[2-(N-méthyl-N-(2-pyridyl)amino)éthoxy]benzyl]thiazolidine-2,4-dione suivant la revendication 1, **caractérisé en ce qu'**il présente un spectre infrarouge suivant la figure 5.

3. Benzènesulfonate de 5-[4-[2-(N-méthyl-N-(2-pyridyl)-amino)éthoxy]benzyl]thiazolidine-2,4-dione suivant la revendication 1 ou 2, **caractérisé en ce qu'**il présente un spectre Raman suivant la figure 6.

4. Benzènesulfonate de 5-[4-[2-(N-méthyl-N-(2-pyridyl)amino)éthoxy]benzyl]thiazolidine-2,4-dione suivant la revendication 1, 2 ou 3, **caractérisé en ce qu'**il présente un diagramme de diffraction des rayons X sur poudre (XRPD) avec les angles et intensités relatives des XRPD caractéristiques :
| Angle | Intensité relative |
|---|---|
| 2-thêta ° | % |
| 6,3 | 3,9 |
| 8,1 | 56,2 |
| 10,3 | 26,7 |
| 12,3 | 9,1 |
| 13,3 | 7,1 |
| 13,7 | 3,6 |
| 14,2 | 13,2 |
| 14,8 | 22,5 |
| 15,1 | 24,6 |
| 16,4 | 52,7 |
| 17,5 | 9,3 |
| 18,5 | 100,0 |
| 19,1 | 35,2 |
| 19,4 | 5,0 |
| 19,7 | 5,2 |
| 20,5 | 6,0 |
| 20,8 | 12,1 |
| 21,2 | 10,4 |
| 21,4 | 9,8 |
| 21,7 | 51,8 |
| 22,7 | 26,3 |
| 22,9 | 35,4 |
| 23,4 | 21,6 |
| 23,6 | 28,4 |
| 24,6 | 25,6 |
| 25,3 | 34,4 |
| 25,6 | 7,0 |
| 26,1 | 11,1 |
| 26,6 | 13,9 |
| 27,0 | 12,2 |
| 27,5 | 18,1 |
| 27,8 | 16,0 |
| 28,6 | 5,8 |
| 29,0 | 3,6 |
| 29,9 | 9,2 |
| 30,6 | 9,5 |
| 31,4 | 24,4 |
| 32,1 | 8,1 |
| 33,2 | 7,7 |
| 33,7 | 12,3 |
| 34,7 | 5,7 |

5. Benzènesulfonate de 5-[4-[2-(N-méthyl-N-(2-pyridyl)amino)éthoxy]benzyl]thiazolidine-2,4-dione suivant l'une quelconque des revendications 1 à 4, **caractérisé en ce qu'**il présente un spectre de ¹³C-RMN à l'état solide suivant la figure 8.

6. Benzènesulfonate de 5-[4-[2-(N-méthyl-N-(2-pyridyl)amino)éthoxy]benzyl]thiazolidine-2,4-dione suivant l'une quelconque des revendications 1 à 5, **caractérisé en ce qu'**il présente :
(i) un spectre infrarouge suivant la figure 5 ; et
(ii) un spectre Raman suivant la figure 6 ; et
(iii) un diagramme de diffraction des rayons X sur poudre (XRPD) avec les angles et intensités relatives des XRPD caractéristiques :
| Angle | Intensité relative |
|---|---|
| 2-thêta ° | % |
| 6,3 | 3,9 |
| 8,1 | 56,2 |
| 10,3 | 26,7 |
| 12,3 | 9,1 |
| 13,3 | 7,1 |
| 13,7 | 3,6 |
| 14,2 | 13,2 |
| 14,8 | 22,5 |
| 15,1 | 24,6 |
| 16,4 | 52,7 |
| 17,5 | 9,3 |
| 18,5 | 100,0 |
| 19,1 | 35,2 |
| 19,4 | 5,0 |
| 19,7 | 5,2 |
| 20,5 | 6,0 |
| 20,8 | 12,1 |
| 21,2 | 10,4 |
| 21,4 | 9,8 |
| 21,7 | 51,8 |
| 22,7 | 26,3 |
| 22,9 | 35,4 |
| 23,4 | 21,6 |
| 23,6 | 28,4 |
| 24,6 | 25,6 |
| 25,3 | 34,4 |
| 25,6 | 7,0 |
| 26,1 | 11,1 |
| 26,6 | 13,9 |
| 27,0 | 12,2 |
| 27,5 | 18,1 |
| 27,8 | 16,0 |
| 28,6 | 5,8 |
| 29,0 | 3,6 |
| 29,9 | 9,2 |
| 30,6 | 9,5 |
| 31,4 | 24,4 |
| 32,1 | 8,1 |
| 33,2 | 7,7 |
| 33,7 | 12,3 |
| 34,7 | 5,7 |
et
(iv) un spectre de ¹³C-RMN à l'état solide suivant la figure 8.

7. Benzènesulfonate de 5-[4- [2- (N-méthyl-N-(2-pyridyl) amino) éthoxy]benzyl] thiazolidine-2,4-dione (Forme III), **caractérisé en ce qu'**il présente :
(i) un spectre infrarouge contenant des pics à 1313, 1163, 706, 568 et 519 cm⁻¹ ; et/ou
(ii) un spectre Raman contenant des pics à 2920, 1442, 1207, 1144, 658 et 308 cm⁻¹ ;
(iii) un diagramme de diffraction des rayons X sur poudre (XRPD) contenant des pics à 11,5, 12,0, 13,1, 17,8 et 18,3 degrés 2θ.

8. Benzènesulfonate de 5-[4-[2-(N-méthyl-N-(2-pyridyl)amino)éthoxy]benzyl]thiazolidine-2,4-dione suivant la revendication 7, **caractérisé en ce qu'**il présente un spectre infrarouge suivant la figure 9.

9. Benzènesulfonate de 5-[4-[2-(N-méthyl-N-(2-pyridyl)amino)éthoxy]benzyl]thiazolidine-2,4-dione suivant la revendication 7 ou 8, **caractérisé en ce qu'**il présente un spectre Raman suivant la figure 10.

10. Benzènesulfonate de 5-[4-[2-(N-méthyl-N-(2-pyridyl)amino)éthoxy]benzyl]thiazolidine-2,4-dione suivant la revendication 7, 8 ou 9, **caractérisé en ce qu'**il présente un diagramme de diffraction des rayons X sur poudre (XRPD) avec les angles et intensités relatives des XRPD caractéristiques :
| Angle | Intensité relative |
|---|---|
| 2-thêta ° | % |
| 9,2 | 2,7 |
| 11,5 | 5,3 |
| 12,0 | 5,4 |
| 13,1 | 31,6 |
| 15,2 | 24,1 |
| 16,4 | 19,6 |
| 17,1 | 18,5 |
| 17,5 | 19,2 |
| 17,8 | 15,4 |
| 18,3 | 38,1 |
| 19,1 | 22,0 |
| 19,4 | 100,0 |
| 19,9 | 6,1 |
| 21,1 | 24,8 |
| 21,8 | 28,3 |
| 22,5 | 23,7 |
| 22,9 | 30,1 |
| 23,2 | 18,6 |
| 23,7 | 27,6 |
| 24,1 | 11,9 |
| 25,1 | 24,8 |
| 25,3 | 21,3 |
| 25,7 | 6,0 |
| 26,4 | 11,7 |
| 27,0 | 17,7 |
| 27,5 | 15,0 |
| 28,1 | 7,0 |
| 29,3 | 20,7 |
| 29,6 | 12,9 |
| 30,3 | 19,4 |
| 30,8 | 9,7 |
| 31,3 | 6,3 |
| 31,8 | 6,3 |
| 32,3 | 7,1 |
| 32,7 | 9,4 |
| 32,9 | 12,3 |
| 33,6 | 7,9 |

11. Benzènesulfonate de 5-[4-[2-(N-méthyl-N-(2-pyridyl)amino)éthoxy]benzyl]thiazolidine-2,4-dione suivant l'une quelconque des revendications 7 à 10, **caractérisé en ce qu'**il présente un spectre de ¹³C-RMN à l'état solide suivant la figure 12.

12. Benzènesulfonate de 5-[4-[2-(N-méthyl-N-(2-pyridyl)amino)éthoxy]benzyl]thiazolidine-2,4-dione suivant l'une quelconque des revendications 7 à 11, **caractérisé en ce qu'**il présente
(i) un spectre infrarouge suivant la figure 9 ; et
(ii) un spectre Raman suivant la figure 10 ; et
(iii) un diagramme de diffraction des rayons X sur poudre (XRPD) avec les angles et intensités relatives des XRPD caractéristiques :
| Angle | Intensité relative |
|---|---|
| 2-thêta ° | % |
| 9,2 | 2,7 |
| 11,5 | 5,3 |
| 12,0 | 5,4 |
| 13,1 | 31,6 |
| 15,2 | 24,1 |
| 16,4 | 19,6 |
| 17,1 | 18,5 |
| 17,5 | 19,2 |
| 17,8 | 15,4 |
| 18,3 | 38,1 |
| 19,1 | 22,0 |
| 19,4 | 100,0 |
| 19,9 | 6,1 |
| 21,1 | 24,8 |
| 21,8 | 28,3 |
| 22,5 | 23,7 |
| 22,9 | 30,1 |
| 23,2 | 18,6 |
| 23,7 | 27,6 |
| 24,1 | 11,9 |
| 25,1 | 24,8 |
| 25,3 | 21,3 |
| 25,7 | 6,0 |
| 26,4 | 11,7 |
| 27,0 | 17,7 |
| 27,5 | 15,0 |
| 28,1 | 7,0 |
| 29,3 | 20,7 |
| 29,6 | 12,9 |
| 30,3 | 19,4 |
| 30,8 | 9,7 |
| 31,3 | 6,3 |
| 31,8 | 6,3 |
| 32,3 | 7,1 |
| 32,7 | 9,4 |
| 32,9 | 12,3 |
| 33,6 | 7,9 |
et
(iv) un spectre de ¹³C-RMN à l'état solide suivant la figure 12.

13. Produit de solvatation de propane-2-ol de benzènesulfonate de 5-[4-[2-(N-méthyl-N-(2-pyridyl)amino)-éthoxy]benzyl]thiazolidine-2,4-dione, **caractérisé en ce qu'**il présente :
(i) un spectre infrarouge contenant un pic à 3541 cm⁻¹ ; et/ou
(ii) un spectre Raman contenant des pics à 893 et 317 cm⁻¹ ;
(iii) un diagramme de diffraction des rayons X sur poudre (XRPD) contenant des pics à 9,5, 13,3, 20,1 et 26,8 degrés 2θ.

14. Benzènesulfonate de 5-[4-[2-(N-méthyl-N-(2-pyridyl)amino)éthoxy]benzyl]thiazolidine-2,4-dione suivant la revendication 13, **caractérisé en ce qu'**il présente un spectre infrarouge suivant la figure 13.

15. Benzènesulfonate de 5-[4-[2-(N-méthyl-N-(2-pyridyl)amino)éthoxy]benzyl]thiazolidine-2,4-dione suivant la revendication 13 ou 14, **caractérisé en ce qu'**il présente un spectre Raman suivant la figure 14.

16. Benzènesulfonate de 5-[4-[2-(N-méthyl-N-(2-pyridyl)amino)éthoxy]benzyl]thiazolidine-2,4-dione suivant la revendication 13, 14 ou 15, **caractérisé en ce qu'**il présente un diagramme de diffraction des rayons X sur poudre (XRPD) avec les angles et intensités relatives des XRPD caractéristiques :
| Angle | Intensité relative |
|---|---|
| 2-thêta ° | % |
| 6,9 | 17,0 |
| 9,0 | 15,5 |
| 9,2 | 10,2 |
| 9,5 | 4,6 |
| 11,7 | 2,7 |
| 13,4 | 10,5 |
| 13,8 | 17,2 |
| 14,8 | 5,8 |
| 16,0 | 8,8 |
| 16,7 | 26,0 |
| 17,2 | 15,7 |
| 17,5 | 6,0 |
| 18,1 | 7,9 |
| 18,6 | 26,6 |
| 18,8 | 29,1 |
| 19,1 | 26,0 |
| 19,4 | 31,4 |
| 19,9 | 23,3 |
| 20,1 | 25,1 |
| 20,8 | 100,0 |
| 21,5 | 16,7 |
| 21,7 | 21,2 |
| 22,5 | 34,1 |
| 23,3 | 11,0 |
| 23,8 | 11,3 |
| 24,1 | 19,6 |
| 24,7 | 17,0 |
| 25,5 | 17,9 |
| 25,7 | 22,8 |
| 26,1 | 17,0 |
| 26,4 | 16,0 |
| 26,8 | 19,9 |
| 27,3 | 15,0 |
| 28,5 | 12,1 |
| 29,7 | 13,5 |
| 30,8 | 10,9 |
| 31,6 | 7,3 |
| 32,5 | 18,9 |
| 32,8 | 18,6 |
| 33,8 | 10,9 |

17. Benzènesulfonate de 5-[4-[2-(N-méthyl-N-(2-pyridyl)amino)éthoxy]benzyl]thiazolidine-2,4-dione suivant l'une quelconque des revendications 13 à 16, **caractérisé en ce qu'**il présente :
(i) un spectre infrarouge suivant la figure 13 ; et
(ii) un spectre Raman suivant la figure 14 ;
(iii) un diagramme de diffraction des rayons X sur poudre (XRPD) avec les angles et intensités relatives des XRPD caractéristiques :
| Angle | Intensité relative |
|---|---|
| 2-thêta ° | % |
| 6,9 | 17,0 |
| 9,0 | 15,5 |
| 9,2 | 10,2 |
| 9,5 | 4,6 |
| 11,7 | 2,7 |
| 13,4 | 10,5 |
| 13,8 | 17,2 |
| 14,8 | 5,8 |
| 16,0 | 8,8 |
| 16,7 | 26,0 |
| 17,2 | 15,7 |
| 17,5 | 6,0 |
| 18,1 | 7,9 |
| 18,6 | 26,6 |
| 18,8 | 29,1 |
| 19,1 | 26,0 |
| 19,4 | 31,4 |
| 19,9 | 23,3 |
| 20,1 | 25,1 |
| 20,8 | 100,0 |
| 21,5 | 16,7 |
| 21,7 | 21,2 |
| 22,5 | 34,1 |
| 23,3 | 11,0 |
| 23,8 | 11,3. |
| 24,1 | 19,6 |
| 24,7 | 17,0 |
| 25,5 | 17,9 |
| 25,7 | 22,8 |
| 26,1 | 17,0 |
| 26,4 | 16,0 |
| 26,8 | 19,9 |
| 27,3 | 15,0 |
| 28,5 | 12,1 |
| 29,7 | 13,5 |
| 30,8 | 10,9 |
| 31,6 | 7,3 |
| 32,5 | 18,9 |
| 32,8 | 18,6 |
| 33,8 | 10,9 |

18. Composé suivant l'une quelconque des revendications 1 à 17, sous forme isolée.

19. Composé suivant l'une quelconque des revendications 1 à 17, sous forme cristalline.

20. Procédé pour la préparation d'un composé suivant l'une quelconque des revendications 1 à 19, **caractérisé en ce que** le 5-[4- [2- (N-méthyl-N-(2-pyridyl)amino)éthoxy]-benzyl]thiazolidine-2,4-dione (Composé I) ou un de ses sels, de préférence dispersé ou dissous dans un solvant convenable, est amené à réagir avec une source d'ion benzènesulfonate, et le sel consistant en benzènesulfonate est recueilli.

21. Forme II ou Forme III ou produit de solvatation de propane-2-ol de 5-[4-[2-(N-méthyl-N-(2-pyridyl)amino)-éthoxy]benzyl]thiazolidine-2,4-dione suivant la revendication 1, 7 ou 13, respectivement, destiné à être utilisé comme substance thérapeutique active.

22. Forme II ou Forme III ou produit de solvatation de propane-2-ol de 5-[4-[2-(N-méthyl-N-(2-pyridyl)amino)-éthoxy]benzyl]thiazolidine-2,4-dione suivant la revendication 1, 7 ou 13, respectivement, destiné à être utilisé dans le traitement et/ou la prophylaxie du diabète sucré, d'affections associées au diabète sucré et de certaines de leurs complications.

23. Utilisation de la Forme II ou de la Forme III ou du produit de solvatation de propane-2-ol de 5-[4-[2-(N-méthyl-N-(2-pyridyl)amino)éthoxy]benzyl]thiazolidine-2,4-dione suivant la revendication 1, 7 ou 13, respectivement, pour la production d'un médicament destiné au traitement et/ou à la prophylaxie du diabète sucré, d'affections associées au diabète sucré et de certaines de leurs complications.
